# EUROPEAN PATENT APPLICATION

(11) **EP 2 314 570 A1**
(43) Date of publication of application: **27.04.2011**
(21) Application number: 09806572.5
(22) Date of filing: 07.08.2009
(51) Int. Cl.: C07C 271/08, A01N 47/24, A01P 3/00

(54) **OXIME ETHER DERIVATIVE AND BACTERICIDE FOR AGRICULTURAL AND HORTICULTURAL USE**

(30) Priority: 11.08.2008 JP 2008207297; 11.08.2008 JP 2008207304; 24.09.2008 JP 2008244957
(71) Applicant: Nippon Soda Co., Ltd., Tokyo 100-8165 (JP)
(72) Inventor: FURUKAWA, Hironori, Odawara-shi Kanagawa 250-0280 (JP); KUWAHARA, Raito, Odawara-shi Kanagawa 250-0280 (JP); KANAZAWA, Jyun, Odawara-shi Kanagawa 250-0280 (JP); SATO, Motoaki, Odawara-shi Kanagawa 250-0280 (JP)
(74) Representative: Wills, Andrew Jonathan
(86) International application number: PCT/JP2009/003812
(87) International publication number: WO 2010/018676

(57) **Abstract**

Provided is a fungicide for agriculture and horticulture containing at least one of oxime ether derivative represented by Formula (I) or a salt thereof (wherein X represents a halogen atom, a C1-C20 alkyl group or the like, R¹ and R² each independently represent a hydrogen atom, a C1-C20 alkyl group, or the like, R³ represents a hydrogen atom, a C1-C20 alkyl group, or the like, R⁴ represents a hydrogen atom, a C1-C20 alkyl group, or the like, R⁵ represents a hydrogen atom or the like, Y represents an oxygen atom or the like, Z represents an oxygen atom or the like, Q represents an aryl group or the like, m represents an integer of 0 to 8, and n represents an integer of 0 to 4).

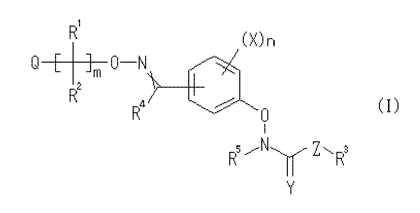

## Description

### TECHNICAL FIELD

The present invention relates to a novel oxime ether derivative and a salt thereof, and a fungicide for agriculture and horticulture containing at least one of these compounds as an active ingredient.
This application claims priority based on Japanese Patent Application No. 2008-207297 and 2008-207304, filed on August 11, 2008, and Japanese Patent Application No. 2008-244957, filed on September 24, 2008, the disclosures of which are incorporated herein by reference.

### BACKGROUND ART

Various agents for controlling diseases of products have been used for preventing disease damage to products in the production of agricultural and horticultural crops, but these are not necessarily satisfactory from the viewpoints of insufficient effectiveness on pests, restriction on their use due to the emergence of disease-causing germs resistant to the agent, phytotoxicity or causing contamination to plants, and toxicity or environmental effects in regard to humans, livestock or fish. Accordingly, there is an increasing need for emergence of chemical agents which reduce these disadvantages and can be safely applied.

Related to the present invention, Patent Document 1 discloses an oxyamine derivative represented by the following formula which has a similar chemical structure to the compound of the present invention, and a fungicide for agriculture and horticulture containing the same as an active ingredient.

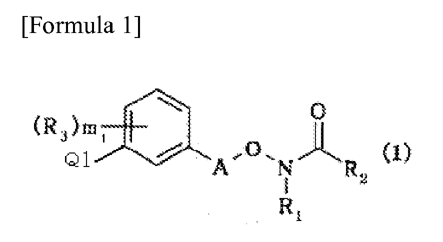

[wherein R₁ represents a hydrogen atom, a C1-C6 alkyl group, a C1-C6 alkylcarbonyl group, or a C1-C6 alkylsulfonyl group. R₂ represents a C1-C6 alkyl group or a C1-C6 alkoxy group. R₃ represents a halogen atom, a C1-C6 alkyl group or a C1-C6 alkoxy group. A represents an optionally branched C1-C6 alkylene group, or a bond. Q1 represents a phenyl group which may be substituted by G, a group represented by Formula (2), or a group represented by Formula (3). m₁ represents an integer of 0 or 1 to 4.

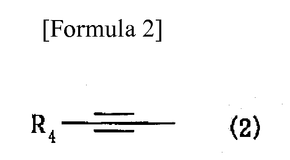

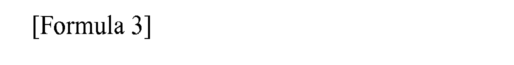

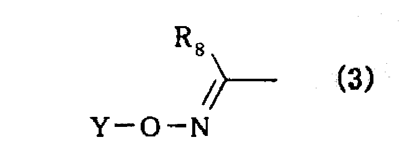

(wherein R₄ represents a hydrogen atom, a C1-C6 alkyl group, a C2-C6 alkenyl group, or SiR₅R₆R₇. R₅ to R₇ each independently represent a C1-C6 alkyl group. R₈ represents a hydrogen atom, a C1-C6 alkyl group, a C1-C6 haloalkyl group, or a phenyl group which may be substituted by G. Y represents a hydrogen atom, a C1-C6 alkyl group, a C3-6 cycloalkyl group, a C3-6 cycloalkyl-C1-C6 alkyl group, a C2-C6 alkenyl group, a C2-C6 alkynyl, or a phenyl C1-C6 alkyl group which may be substituted by G)
G represents a halogen atom, a C1-C6 alkyl group, a C1-C6 alkoxy group, a C1-C6 haloalkyl group or a C1-C6 haloalkoxy group, and G may be identical or different and two to five thereof may be substituted]

However, regarding the case where Q1 is represented by Formula (3), there is no specific mention or specific compounds in the Preparation Examples and Examples of this document.

### PRIOR ART DOCUMENTS

### PATENT DOCUMENTS

[Patent document 1] JP-A-2004-168683

### DISCLOSURE OF INVENTION

### PROBLEMS TO BE SOLVED BY THE INVENTION

The present invention has been made to solve the above problems and it is an object of the present invention to provide a novel oxime ether derivative useful as an active ingredient of fungicides for agriculture and horticulture, which exhibits potent effects and can be safely thus used, and a salt thereof and a fungicide for agriculture and horticulture containing at least one of these compounds as an active ingredient.

### MEANS TO SOLVE THE PROBLEMS

In order to solve the technical problem, in accordance with a first aspect of the present invention, provided is an oxime ether derivative or a salt thereof represented by Formula (I) below:

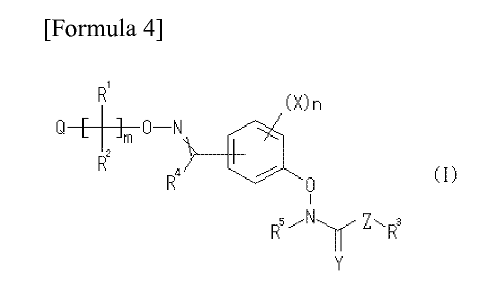

[wherein
X represents a halogen atom, a C1-C20 alkyl group or a C1-C20 alkoxy group, and in the case where n is 2 or more, adjacent X may be bonded together to form a ring.
R¹ and R² each independently represent a hydrogen atom, a halogen atom, a C1-C20 alkyl group or a C3-C10 cycloalkyl group. R¹ and R² may be bonded together to form a ring.
R³ represents a hydrogen atom, a C1-C20 alkyl group, a C2-C20 alkenyl group, a C2-C20 alkynyl group or a C3-C10 cycloalkyl group.
R⁴ represents a hydrogen atom, a C1-C20 alkyl group, a C2-C20 alkenyl group, a C2-C20 alkynyl group, a C3-C10 cycloalkyl group, a cyano group or an amino group.
R⁵ represents a hydrogen atom, a C1-C20 alkyl group, a C2-C20 alkenyl group, a C2-C20 alkynyl group, an acyl group, a C1-C20 alkylsulfonyl group, or a C1-C20 arylsulfonyl group.
Y represents an oxygen atom or a sulfur atom,
Z represents a single bond, an oxygen atom, a sulfur atom, or a group represented by NR⁶ (in which R⁶ represents a hydrogen atom or a C1-C30 alkyl group).
Q represents a C1-C20 alkyl group, an aryl group, a saturated heterocyclic ring, or a group represented by Formula (III) or Formula (IV).

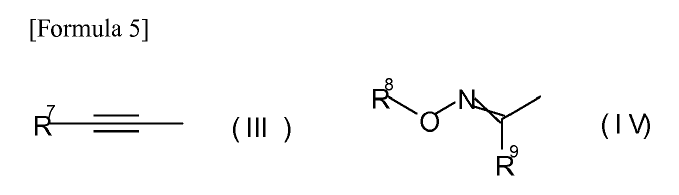

(wherein
R⁷ represents a hydrogen atom, a halogen atom or a C1-C20 alkyl group.
R⁸ represents a hydrogen atom or a C1-C20 alkyl group.
R⁹ represents a hydrogen atom or a C1-C20 alkyl group.)
m represents an integer of 0 to 8, and in the case where m is 2 or more, each of R¹ and R² may independently be identical or different.
n represents an integer of 0 to 4, and in the case where n is 2 or more, may be identical or different.]

The oxime ether derivative or a salt thereof represented by Formula (I) is preferably an oxime ether derivative or a salt thereof represented by Formula (V) or (VI), more preferably, an oxime ether derivative or a salt thereof represented by Formula (VI).

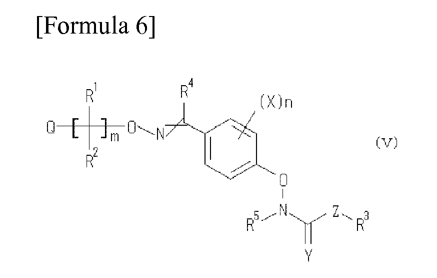

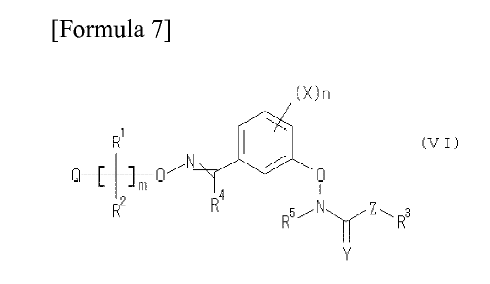

(wherein X, R¹ to R⁵, Y, Z, Q, m and n are as defined above) In accordance with a second aspect of the present invention, provided is a fungicide for agriculture and horticulture containing at least one of the oxime ether derivative or a salt thereof of the present invention as an active ingredient.

### EFFECT OF THE INVENTION

The oxime ether derivative and a salt thereof of the present invention are novel compounds, can be industrially advantageously prepared, and are useful as an active ingredient of a fungicide for agriculture and horticulture which exhibits potent effects and can thus be safely used.
The fungicide for agriculture and horticulture of the present invention exhibits superior insect control, is free of phytotoxicity or causing contamination to plants, and has low toxicity or environmental effects in regard to humans, livestock or fish.

### BEST MODE FOR CARRYING OUT THE INVENTION

Hereinafter, the present invention will be illustrated in detail.

### 1) Oxime ether derivative represented by Formula (I) or a salt thereof

A first aspect of the present invention is an oxime ether derivative represented by Formula (I) or a salt thereof.

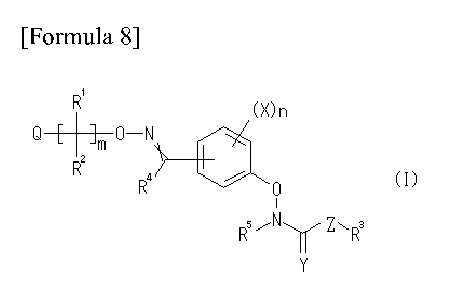

[wherein
X represents a halogen atom, a C1-C20 alkyl group or a C1-C20 alkoxy group, and in the case where n is 2 or more, adjacent X may be bonded together to form a ring;
R¹ and R² each independently represent a hydrogen atom, a halogen atom, a C1-C20 alkyl group or a C3-C10 cycloalkyl group. R¹ and R² may be bonded together to form a ring;
R³ represents a hydrogen atom, a C1-C20 alkyl group, a C2-C20 alkenyl group, a C2-C20 alkynyl group or a C3-C10 cycloalkyl group;
R⁴ represents a hydrogen atom, a C1-C20 alkyl group, a C2-C20 alkenyl group, a C2-C20 alkynyl group, a C3-C10 cycloalkyl group, a cyano group or an amino group;
R⁵ represents a hydrogen atom, a C1-C20 alkyl group, a C2-C20 alkenyl group, a C2-C20 alkynyl group, an acyl group, a C1-C20 alkylsulfonyl group, or a C1-C20 arylsulfonyl group;
Y represents an oxygen atom or a sulfur atom;
Z represents a single bond, an oxygen atom, a sulfur atom, or a group represented by NR⁶ (in which R⁶ represents a hydrogen atom or a C1-C30 alkyl group; and
Q represents a C1-C20 alkyl group, an aryl group, a saturated heterocyclic ring, or a group represented by Formula (III) or Formula (IV).

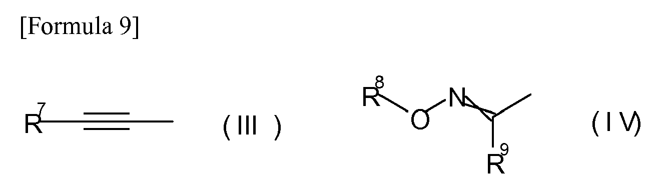

[wherein
R⁷ represents a hydrogen atom, a halogen atom, or a C1-C20 alkyl group;
R⁸ represents a hydrogen atom or a C1-C20 alkyl group;
R⁹ represents a hydrogen atom or a C1-C20 alkyl group;
m represents an integer of 0 to 8, and in the case where m is 2 or more, each of R¹ and R² may independently be identical or different; and
n represents an integer of 0 to 4, and in the case where n is 2 or more, X may be identical or different.]

In addition, in the specification, all of groups, which can chemically have a substituent group such as "C1-C20 alkyl group" or "C1-C20 alkoxy group," include groups having a substituent group. In addition, the number of carbon atoms regarding the term "C1-C20 alkyl group" excludes the number of carbon atoms of the substituent group. For example, in the case of a butyl group having an ethoxy group as a substituent group, the butyl group belongs to a C4 alkyl group.
Any substituent group may be used without particular limitation as long as it is chemically acceptable and can exert the effects of the present invention, and examples thereof are as follows. These substituent groups may be further substituted by the following exemplary substituent groups:

halogen atoms such as a fluorine atom, a chlorine atom, a bromine atom and an iodine atom;
alkyl groups such as a methyl group, an ethyl group, an n-propyl group, an i-propyl group, an n-butyl group, an s-butyl group, an i-butyl group, a t-butyl group, a n-pentyl group and a n-hexyl group, preferably, C1-C6 alkyl groups;
cycloalkyl groups such as a cyclopropyl group, a cyclobutyl group, a cyclopentyl group, a cyclohexyl group and a cycloheptyl group, preferably, C3-C8 cycloalkyl groups;
alkenyl groups such as a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group and a cinnamyl group, preferably, C2-C6 alkenyl groups;
cycloalkenyl groups such as a 2-cyclopropenyl group, a 2-cyclopentenyl group, a 3-cyclohexenyl group, and a 4-cyclooctenyl group, preferably, C3-C8 cycloalkenyl groups;
alkynyl such as an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group, preferably C2-C6 alkynyl groups;
alkoxy groups such as a methoxy group, an ethoxy group, an n-propoxy, an isopropoxy, an n-butoxy group, an s-butoxy group, an isobutoxy group, and a t-butoxy group, preferably C1-C6 alkoxy groups;
alkenyloxy groups such as a vinyloxy group, an allyloxy group, a propenyloxy group, and a butenyloxy group, preferably C2-C6 alkenyloxy groups;
alkynyloxy groups such as an ethynyloxy group and a propargyloxy group, preferably C2-C6 alkynyloxy groups;
aryl groups such as a phenyl group, a 1-naphthyl group, and a 2-naphthyl group, preferably C6-C10 aryl groups;
aryloxy groups such as a phenoxy group, and a 1-naphthoxy group, preferably C6-C10 aryloxy groups;
aralkyl groups such as a benzyl group and a phenethyl group; preferably C7-C11 aralkyl groups;
aralkyloxy groups such as a benzyloxy group and a phenethyloxy group, preferably C7-12 aralkyloxy groups;
acyl groups such as a formyl group, an acetyl group, a propionyl group, a benzoyl group and a cyclohexylcarbonyl group, preferably C1-C7 acyl groups;
acyloxy groups such as a formyloxy group, an acetyloxy group, a propionyloxy group, a benzoyloxy group and a cyclohexylcarbonyloxy group, preferably C1-C7 acyloxy groups;
alkoxycarbonyl groups such as a methoxycarbonyl group, an ethoxycarbonyl group, an n-propoxycarbonyl group, an isopropoxycarbonyl group, an n-butoxycarbonyl group, a t-butoxycarbonyl group, preferably C1-C6 alkoxycarbonyl groups;
a carboxyl group;
a hydroxy group;

haloalkyl groups such as a chloromethyl group, a chloroethyl group, a 1,2-dichloro-n-propyl group, a 1-fluoro-n-butyl group, a perfluoro-n-pentyl group, preferably C1-C6 haloalkyl groups;
haloalkenyl groups such as a 2-chloro-1-propenyl group and a 2-fluoro-1-butenyl group, preferably C2-C6 haloalkenyl groups;
haloalkynyl groups such as a 4,4-dichloro-1-butynyl group, a 4-fluoro-1-pentynyl group, a 5-bromo-2-pentynyl group, preferably C2-C6 haloalkynyl groups;
haloalkoxy groups such as a 2-chloro-n-propoxy group and a 2,3-dichlorobutoxy group, preferably C1-C6 haloalkoxy groups;
haloalkenyloxy groups such as a 2-chloropropenyloxy group and a 3-bromobutenyloxy group, preferably C2-C6 haloalkenyloxy groups;
haloaryl groups such as a 4-chlorophenyl group, a 4-fluorophenyl group, and a 2,4-dichlorophenyl group, preferably C6-C10 haloaryl groups;
haloaryloxy groups such as a 4-fluorophenyloxy group and a 4-chloro-1-naphthoxy group, preferably C6-C10 haloaryloxy groups;
halogen-substituted acyl groups such as a chloroacetyl group, a trifluoroacetyl group, a trichloroacetyl group and a 4-chlorobenzoyl group;

a cyano group; an isocyano group; a nitro group; an isocyanato group; a cyanato group; an amino group;
alkyl amino groups such as a methylamino group, a dimethylamino group, a diethylamino group;
arylamino groups such as an anilino group, a naphthylamino group and an anthracenylamino group;
aralkylamino groups such as a benzylamino group and a phenylethylamino group;
alkylsulfonylamino groups such as a methylsulfonylamino group, an ethylsulfonylamino group, an n-propylsulfonylamino group, an isopropylsulfonylamino group, an n-butylsulfonylamino group and a t-butylsulfonyl amino group;
arylsulfonylamino groups such as a phenylsulfonylamino group;
heteroarylsulfonylamino groups such as a piperadinyl sulfonylamino group;
acylamino groups such as a formylamino group, an acetylamino group, a propanoylamino group, a butyrylamino group, an isopropylcarbonylamino group and a benzoylamino group;
alkoxycarbonylamino groups such as a methoxycarbonylamino group, an ethoxycarbonylamino group, an n-propoxycarbonylamino group and an isopropoxycarbonyl amino group;
haloalkylsulfonylamino groups such as a fluoromethylsulfonylamino group, a chloromethylsulfonylamino group, a bromomethylsulfonylamino group, a difluoromethylsulfonylamino group, a dichloromethylsulfonylamino group, a 1,1-difluoroethylsulfonylamino group, a trifluoromethylsulfonylamino group, a 1,1,1-trifluoroethylsulfonylamino group and a pentafluoroethylsulfonylamino group;
bis(alkylsulfonyl)amino groups such as a bis(methylsulfonyl)amino group, a bis(ethylsulfonyl)amino group, an (ethylsulfonyl)(methylsulfonyl)amino group, a bis(n-propylsulfonyl)amino group, a bis(isopropylsulfonyl)amino group, a bis(n-butylsulfonyl)amino group and a bis(t-butylsulfonyl)amino group;
bis(haloalkylsulfonyl)amino groups such as a bis(fluoromethylsulfonyl)amino group, a bis(chloromethylsulfonyl)amino group, a bis(bromomethylsulfonyl)amino group, a bis(difluoromethylsulfonyl)amino group, a bis(dichloromethylsulfonyl)amino group, a bis(1,1-difluoroethylsulfonyl)amino group, a bis(trifluoromethylsulfonyl)amino group, a bis(1,1,1-trifluoroethylsulfonyl)amino group, and a bis(pentafluoroethylsulfonyl)amino group;
an unsubstituted or substituted hydrazino group such as a hydrazino group, a N'-phenylhydrazino group, a N'-methoxycarbonylhydrazino group, a N'-acetylhydrazino group, and a N'-methylhydrazino group;
aminocarbonyl groups having an unsubstituted or substituted group such as an aminocarbonyl group, a dimethylaminocarbonyl group, a phenylaminocarbonyl group, and a N-phenyl-N-methylaminocarbonyl group;
hydrazinocarbonyl groups having an unsubstituted or substituted group such as a hydrazinocarbonyl group, a N'-methylhydrazinocarbonyl group, and a N'-phenylhydrazinocarbonyl group;
N-unsubstituted or N-substituted iminoalkyl groups such as an N-methyliminomethyl group, a 1-N-phenyliminoethyl group, an N-hydroxyiminomethyl group, and an N-methoxyiminomethyl group.

a thiol group;
an isothiocyanato group;
a thiocyanato group;
alkylthio groups such as a methylthio group, an ethylthio group, an n-propylthio group, an isopropylthio group, an n-butylthio group, an isobutylthio group, an s-butylthio group, and a t-butylthio group;
alkenylthio groups such as a vinylthio group and an allylthio group;
alkynylthio groups such as an ethynylthio group and a propargylthio group;
arylthio groups such as a phenylthio group and a naphthylthio group;
heteroarylthio groups such as a 2-piperidylthio group and a 3-pyridazylthio group;
aralkylthio groups such as a benzylthio group and a phenethylthio group;
alkylthiocarbonyl groups such as a methylthiocarbonyl group, an ethylthiocarbonyl group, a n-propylthiocarbonyl group, an isopropylthiocarbonyl group, a n-butylthiocarbonyl group, an isobutylthiocarbonyl group, a s-butylthiocarbonyl group and a t-butylthiocarbonyl group;
alkylsulfinyl groups such as methylsulfinyl, ethylsulfinyl, and t-butylsulfinyl;
alkenylsulfinyl groups such as an allylsulfinyl group;
alkynylsulfinyl groups such as a propargylsulfinyl group;
arylsulfinyl groups such as a phenylsulfinyl group;
heteroarylsulfinyl such as 2-pyridylsulfinyl and 3-pyridylsulfinyl groups;
aralkylsulfinyl groups such as benzylsulfinyl and phenethylsulfinyl groups;
alkylsulfonyl groups such as a methylsulfonyl group, an ethylsulfonyl group, and a t-butylsulfonyl group;
alkenylsulfonyl groups such as an allylsulfonyl group;
alkynylsulfonyl groups such as a propargylsulfonyl group;
arylsulfonyl groups such as a phenylsulfonyl group;
heteroarylsulfonyl groups such as a 2-pyridylsulfonyl group and a 3-pyridylsulfonyl group;
aralkylsulfonyl groups such as a benzylsulfonyl group and a phenethylsulfonyl group;

unsaturated heterocyclic 5-membered groups such as a furan-2-yl group, a furan-3-yl group, a thiophen-2-yl group, a thiophen-3-yl group, a pyrrol-2-yl group, a pyrrol-3-yl group, an oxazol-2-yl group, an oxazol-4-yl group, an oxazol-5-yl group, a thiazol-2-yl group, a thiazol-4-yl group, a thiazol-5-yl group, an isoxazol-3-yl group, an isoxazol-4-yl group, an isoxazol-5-yl group, an isothiazol-3-yl group, an isothiazol-4-yl group, an isothiazol-5-yl group, an imidazol-2-yl group, an imidazol-4-yl group, an imidazol-5-yl group, a pyrazol-3-yl group, a pyrazol-4-yl group, a pyrazol-5-yl group, a 1,3,4-oxadiazol-2-yl group, a 1,3,4-thiadiazol-2-yl group, a 1,2,3-triazol-4-yl group, a 1,2,4-triazol-3-yl group and a 1,2,4-triazol-5-yl group;

unsaturated heterocyclic 6-membered groups such as a pyridin-2-yl group, a pyridin-3-yl group, a pyridin-4-yl group, a 5-chloro-3-pyridyl group, a 3-trifluoromethyl-2-pyridyl group, a pyridazin-3-yl group, a pyridazin-4-yl group, a pyrazin-2-yl group, a pyrimidin-2-yl group, a pyrimidin-4-yl group, a pyrimidin-5-yl group, a 1,3,5-triazin-2-yl group and a 1,2,4-triazin-3-yl group;
saturated heterocyclic groups such as a tetrahydrofuran-2-yl group, a tetrahydrapyran-4-yl group, a piperidin-3-yl group, a pyrrolidin-2-yl group, a morpholino group, a piperidino group and a N-methyl piperadinyl group; and
heterocyclic oxy groups such as a 2-pyridyloxy group and a 3-oxazolyloxy group.

(X)
In Formula (I), X represents a halogen atom, a C1-C20 alkyl group or a C1-C20 alkoxy group.
n represents an integer of 0 to 4, preferably an integer of 0 to 2. In the case where n is 2 or more, X may be identical or different, two or more adjacent X may be bonded together to form a ring.

Regarding X, examples of the halogen atom include a fluorine atom, a chlorine atom and a bromine atom.

Regarding X, examples of the C1-C20 alkyl group include a methyl group, an ethyl group, an n-propyl group, an i-propyl group, an n-butyl group, an i-butyl group, an s-butyl group, a t-butyl group, an n-pentyl group, an n-hexyl group, an n-heptyl group, an n-octyl group, an n-nonyl group, an i-nonyl group, a decyl group, a lauryl group, a tridecyl group, a myristyl group, a pentadecyl group, a palmityl group, a heptadecyl group and a stearyl group. Preferred is a C1-C6 alkyl group.

Regarding X, examples of the C1-C20 alkoxy group include a methoxy group, an ethoxy group, an n-propoxy group, an i-propoxy group, an n-butoxy group, an i-butoxy group, an s-butoxy group, a t-butoxy group, an n-pentyloxy group, an n-hexyloxy group, an n-heptyloxy group, an i-heptyloxy group, a t-heptyloxy group, an n-octyloxy group, an i-octyloxy group, a t-octyloxy group, a 2-ethylhexyloxy group, a nonyloxy group, a decyloxy group, a lauryloxy group, a tridecyloxy group, a myristyloxy group, a pentadecyloxy group, a palmityloxy group, a heptadecyloxy group, and a stearyloxy group. Preferred is a C1-C6 alkoxy group.

The C1-C20 alkyl group and C1-C20 alkoxy group of X may have one or more substituent groups, and in the case where these groups have two or more substituent groups, the substituent groups may be identical or different and examples of the substituent group are described above.

In the case where the C1-C20 alkyl group or C1-C20 alkoxy group of X has a substituent group, the substituent group is preferably a halogen atom. That is, in the case where X has a substituent group, X is preferably a C1-C20 haloalkyl group or C1-C20 haloalkoxy group.

Examples of the C1-C20 haloalkyl group include a fluoromethyl group, a chloromethyl group, a bromo methyl group, a difluoromethyl group, a dichloromethyl group, a dibromomethyl group, a trifluoromethyl group, a trichloromethyl group, a tribromo methyl group, a 2,2,2-trifluoroethyl group, a 2,2,2-trichloroethyl group and a pentafluoroethyl group.

Examples of the C1-C20 haloalkoxy group include a chloromethoxy group, a dichloromethoxy group, a trichloromethoxy group, a trifluoromethoxy group, a 1-fluoroethoxy group, a 1,1-difluoroethoxy group, a 2,2,2-trifluoroethoxy group and a pentafluoroethoxy group.

(R¹, R²)
R¹ and R² each independently represent a hydrogen atom, a halogen atom, a C1-C20 alkyl group or a C3-C10 cycloalkyl group. R¹ and R² may be bonded together to form a ring.
m represents an integer of 0 to 8, m is preferably an integer of 1 to 8, more preferably, an integer of 1 to 3.
In the case where m is 2 or more, each of R¹ and R² may independently be identical or different.

Specific examples of the halogen atom and C1-C20 alkyl group regarding R¹ and R² are the same as the examples of halogen atom and C1-C20 alkyl group regarding X.

The C3-C10 cycloalkyl group of R¹, R² is a monocyclic or polycyclic alkyl group, and examples thereof include a cyclopropyl group, a cyclobutyl group, a cyclopentyl group, a cyclohexyl group, a cycloheptyl group, a cyclooctyl group, a cyclodecyl group and a bicyclo[3. 2. 1]octyl group.

The C1-C20 alkyl group and C3-C10 cycloalkyl group of R¹ and R² may have one or more substituent groups, in the case where these groups have two or more substituent groups, the substituent groups may be identical or different and examples of the substituent group are described above.

Among the substituent groups, the substituent group of C1-C20 alkyl group is preferably a halogen atom, the substituent group of C3-C10 cycloalkyl group is preferably a halogen atom, a C1-C6 alkyl group, a C1-C6 alkoxy group, a nitro group and/or a cyano group.

In addition, in the case where R¹ and R² are bonded together to form a ring, examples of the ring include C3-C10 cycloalkane rings such as a cyclopropane ring, a cyclopentane ring, and a cyclohexane ring.

(R³)
R³ represents a hydrogen atom, a C1-C20 alkyl group, a C2-C20 alkenyl group, a C2-C20 alkynyl group or a C3-C10 cycloalkyl group.

Examples of the C1-C20 alkyl group regarding R³ are the same as examples of the C1-C20 alkyl group regarding X, and specific examples of the C3-C10 cycloalkyl group are the same as the examples of the C3-C10 cycloalkyl group regarding R¹ and R².

Examples of the C2-C20 alkenyl group regarding R³ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, a 5-hexenyl group, a heptenyl group, an octenyl group, a decenyl group, a pentadecenyl group and an icosenyl group. Preferred is a C2-C6 alkenyl group.

Examples of the C2-C20 alkynyl group include an ethynyl group, a 1-propynyl group, a propargyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, a 1,1-dimethyl-2-butynyl group, a heptynyl group, an octynyl group, a decynyl group, a pentadecynyl group and an icosinyl group. Preferred is a C2-C6 alkynyl group.

The C1-C20 alkyl group, C2-C20 alkenyl group, C2-C20 alkynyl and C3-C10 cycloalkyl group of R³ may have one or more substituent groups, and in the case where these groups have two or more substituent groups, the substituent groups may be identical or different.
Examples of the substituent group are described above. Among the substituent groups, the substituent group of the C1-C20 alkyl group is preferably a halogen atom, the substituent group of the C3-C10 cycloalkyl group is preferably a halogen atom, a C1-C6 alkyl group, a C1-C6 alkoxy group, a nitro group, and/or a cyano group.

In the case where R³ is a C2-C20 haloalkenyl group in which the C2-C20 alkenyl group is substituted by a halogen atom, specific examples of the C2-C20 haloalkenyl group include a 3-chloro-2-propenyl group, a 4-chloro-2-butenyl group, a 4,4-dichloro-3-butenyl group, a 4,4-difluoro3-butenyl group and a 3,3-dichloro-2-propenyl group.

In the case where R³ is a C2-C20 haloalkynyl group in which the C2-C20 alkynyl group is substituted by a halogen atom, examples of the C2-C20 haloalkynyl group include a 3-chloro-1-propynyl group, a 3-chloro-1-butynyl group, a 3-bromo-1-butynyl group, a 3-bromo-2-propynyl group and a 3-iodo-2-propynyl group.

(R⁴)
R⁴ represents a hydrogen atom, a C1-C20 alkyl group, a C2-C20 alkenyl group, a C2-C20 alkynyl, a C3-C10 cycloalkyl group, a cyano group, or an amino group. Of these, R⁴ is preferably a hydrogen atom, a C1-C20 alkyl group, a C2-C20 alkenyl group, a C2-C20 alkynyl, a cyano group or an amino group.

Specific examples of the C1-C20 alkyl group of R⁴ are the same as specific examples of C1-C20 alkyl group of X and specific examples of the C3-C10 cycloalkyl group are the same as specific examples of C3-C10 cycloalkyl group of R¹ and R².
Specific examples of the C2-C20 alkenyl group and C2-C20 alkynyl group of R⁴ are the same as specific examples of the C2-C20 alkenyl group and C2-C20 alkynyl group of R³.

These groups may be unsubstituted or substituted, and examples of the substituent group are the same as those exemplified above.
In the case where these groups have a substituent group, the substituent group of the C1-C20 alkyl group, the C2-C20 alkenyl group and the C2-C20 alkynyl is preferably a halogen atom. That is, preferred are a C1-C20 haloalkyl group, a C2-C20 haloalkenyl group and C2-C20 haloalkynyl. Meanwhile, the substituent group of the C3-C10 cycloalkyl group is preferably a halogen atom, a C1-C6 alkyl group, a C1-C6 alkoxy group, a nitro group, and/or a cyano group.

In the case where R⁴ is an amino group, the amino group may be unsubstituted or have a substituent group and examples of the substituent group are the same as those exemplified above.

Specific examples of preferred amino groups having a substituent group include: mono C1-C6 alkylamino groups such as methylamino groups, ethylamino groups and i-propylamino groups; di C1-C6 alkylamino groups such as dimethylamino groups and diethylamino groups; acylamino groups such as acetylamino groups and benzoylamino groups; phenylamino groups optionally having a substituent group such as phenylamino groups and 4-methylphenylamino groups.
In addition, in the case where R⁴ is an amino group, the amino group may be a tautomer represented by the following Formula:

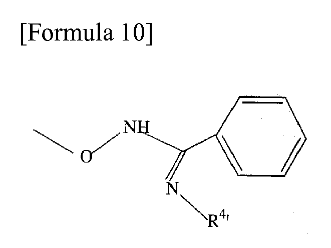

(wherein N-R⁴, represents an amino group as exemplified above.)

(R⁵)
R⁵ represents a hydrogen atom, a C1-C20 alkyl group, a C2-C20 alkenyl group, C2-C20 alkynyl, an acyl group, a C1-C20 alkylsulfonyl group or a C1-C20 arylsulfonyl group.

Specific examples of the C1-C20 alkyl group of R⁵ are the same as the specific examples of the C1-C20 alkyl group of X. Specific examples of the C2-C20 alkenyl group and the C2-C20 alkynyl group are the same as specific examples of the C2-C20 alkenyl group and the C2-C20 alkynyl group of R³.

An acyl group refers to a group such as a hydrogen atom, an alkyl group, an alkenyl group, an alkynyl group, an aryl group or a heteroaryl group bonded to a carbonyl group. Examples of the acyl group include a formyl group; alkylcarbonyl groups such as an acetyl group, a propionyl group, a butyloyl group, a pentanoyl group, a hexanoyl group, a heptanoyl group, an octanoyl group, a nonanoyl group, a decanoyl group, a 3-methylnonanoyl group, a 8-methylnonanoyl group, a 3-ethyloctanoyl group, a 3,7-dimethyloctanoyl group, an undecanoyl group, a dodecanoyl group, a tridecanoyl group, a tetradecanoyl group, a pentadecanoyl group, a hexadecanoyl group, a 1-methylpentadecanoyl group, a 14-methylpentadecanoyl group, a 13,13-dimethyltetradecanoyl group, a heptadecanoyl group, a 15-methylhexadecanoyl group, an octadecanoyl group, a 1-methylheptadecanoyl group, a nonadecanoyl group, an icosanoyl group and a henicosanoyl group;
alkenylcarbonyl groups such as an acryloyl group, a methacryloyl group, an allylcarbonyl group and a cinnamoyl group;
an ethynylcarbonyl group and a propinylcarbonyl group;
arylcarbonyl groups such as a benzoyl group, a naphthylcarbonyl group, a biphenylcarbonyl group and an anthranylcarbonyl group; and
heteroarylcarbonyl groups such as a 2-pyridylcarbonyl group and a thienylcarbonyl group. Of these, preferred is a C1-C20 acyl group (containing a carbonyl group), more preferred is a C1-C7 acyl group.

Examples of the C1-C20 alkylsulfonyl group include a methylsulfonyl group, an ethylsulfonyl group, an n-propylsulfonyl group and an i-propylsulfonyl group. Preferred is a C1-C6 alkylsulfonyl group.
Examples of the C1-C20 arylsulfonyl group include a phenylsulfonyl group, a naphthylsulfonyl group and an anthracenylsulfonyl group. Of these, preferred is a C1-C10 arylsulfonyl group.

The C1-C20 alkyl group, the acyl group, the C1-C20 alkylsulfonyl group and the C1-C20 arylsulfonyl group may have a substituent group, and examples of the substituent group are the same as those exemplified above.

Among the substituent groups, the substituent group of the C1-C20 alkyl group is preferably an alkoxy group, and examples of the alkoxy-substituted alkyl group include a methoxymethyl group, an ethoxymethyl group, an i-propoxymethyl group, a 1-methoxyethyl group, a 2-methoxyethyl group, a 1-ethoxyethyl group, a 2-ethoxyethyl group, a 1-methoxy-n-propyl group, a 2-methoxy-n-propyl group and a 3-methoxy-n-propyl group.
The substituent group of the acyl group and phenylsulfonyl group is preferably an alkyl group or a halogen atom, examples of the substituted acyl group include a trifluoroacetyl group, a trichloroacetyl group, a 4-methylbenzoyl group and a 2-chlorobenzoyl group, and examples of the substituted phenylsulfonyl group include a 4-methylphenylsulfonyl group, a 2-chlorophenylsulfonyl group and a 2,4-dimethylphenylsulfonyl group.

(Y, Z, R⁶)
Y represents an oxygen atom or a sulfur atom.
Z represents a single bond, an oxygen atom, a sulfur atom, or a group represented by NR⁶.
R⁶ represents a hydrogen atom or a C1-C30 alkyl group. Examples of the C1-C30 alkyl group include, in addition to examples of the C1-C20 alkyl group regarding X, a docosyl group, a tricosyl group, a pentacosyl group and an octacosyl group. Preferred is a C1-C6 alkyl group. The C1-C30 alkyl group may be unsubstituted or substituted and examples of the substituent group are the same as exemplified above.

(Q)
Q represents a C1-C20 alkyl group, an aryl group, a saturated heterocyclic group, or a group represented by the following Formula (III) or Formula (IV).

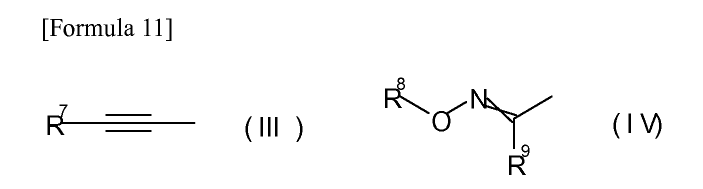

(wherein R⁷ represents a hydrogen atom, a halogen atom or a C1-C20 alkyl group, R⁸ represents a hydrogen atom or a C1-C20 alkyl group and R⁹ represents a hydrogen atom or a C1-C20 alkyl group.)
Of these, Q preferably represents an aryl group or a group represented by Formula (III).

Specific examples of C1-C20 alkyl group regarding Q are the same as specific examples of C1-C20 alkyl group regarding X. These groups may be unsubstituted or substituted, and examples of the substituent group are the same as those exemplified above. Of these, preferred is a halogen atom, and more preferred is a C1-C20 haloalkyl group.

The aryl group of Q refers to a monocyclic or polycyclic aryl group. In the case of a polycyclic aryl group, the polycyclic aryl group may be entirely or partially unsaturated. Examples of the aryl group include a phenyl group, a naphthyl group, an anthracen-1-yl group, a phenanthren-1-yl group, an indenyl group, an indanyl group, and a tetralinyl group. Preferred is a C6-C10 aryl group.

Examples of the substituent group of aryl group include, in addition to the substituent groups as exemplified above, optionally substituted imino groups. Examples of substituent groups of optionally substituted imino groups include the substituent groups as exemplified above. Specific examples of optionally substituted imino groups include an ethylidenamino group, a 1-methylpropylidenamino group, a 1,3-dimethylbutylidenaminogroup, a 1-methylethylidenamino group, a 4-N,N-dimethylaminobenzylidenamino group, a cyclohexylidenamino group and a methoxyiminoacetyl group.
Examples of preferred substituent groups include halogen atoms, alkoxy groups, alkylthio groups, alkylsulfonyl groups, alkylsulfoxy groups, cyano groups and nitro groups. Respective specific examples are the same as those of the substituent groups and are more preferably groups substituted by at least one from the group consisting of fluorine atoms and fluorine atom-containing groups.
The number of fluorine atoms and fluorine atom-containing substituent groups substituted in an aryl group is not particularly limited, and in the case where the number is two or more, the substituent groups may be identical or different.

Examples of the fluorine atom-containing substituent group include fluorine-substituted alkyl groups, fluorine-substituted alkenyl groups, fluorine-substituted alkynyl groups, fluorine-substituted cycloalkyl groups, fluorine-substituted cycloalkenyl groups, fluorine-substituted cycloalkynyl groups, fluorine-substituted alkoxy groups, fluorine-substituted alkenyloxy groups, fluorine-substituted alkynyloxy groups and fluorine-substituted acyl groups. Preferred are fluorine-substituted alkyl groups and fluorine-substituted alkoxy groups.

Examples of fluorine-substituted alkyl groups include fluoromethyl groups, difluoromethyl groups, perfluoromethyl groups, 1-fluoroethyl groups, 1,2-difluoroethyl groups, 2,2-difluoroethyl groups, 1,2,2-trifluoroethyl groups, 2,2,2-trifluoroethyl groups, pentafluoroethyl groups and perfluorobutyl groups.

Examples of the fluorine-substituted alkoxy group include fluoromethoxy groups, difluoromethoxy groups, trifluoromethoxy groups, 1-fluoroethoxy groups, 1,1-difluoroethoxy groups, 2,2,2-trifluoroethoxy groups, pentafluoroethoxy groups, and perfluorobutoxy groups.

Specific examples of saturated heterocyclic groups of Q include 5-membered saturated heterocyclic groups such as tetrahydrofuran-2-yl groups, tetrahydrofuran-3-yl groups, pyrrolidin-1-yl groups, pyrrolidin-2-yl groups, pyrrolidin-3-yl groups, tetrahydrothiophen-2-yl groups, tetrahydrothiophen-3-yl groups; and 6-membered saturated heterocyclic groups such as tetrahydropyran-2-yl groups, tetrahydropyran-3-yl groups, tetrahydropyran-4-yl groups, piperidin-1-yl groups, piperidin-2-yl groups, piperidin-3-yl groups, piperidin-4-yl groups, piperazin-1-yl groups, piperazin-2-yl groups, piperazin-3-yl groups, morpholin-2-yl groups, morpholin-3-yl groups, and morpholin-4-yl groups.
Examples of the substituent group of saturated heterocyclic groups of Q include examples of the substituent group of X. Of these, preferred is a piperidin-2-yl group.

The halogen atom and C1-C20 alkyl group of R⁷ in Formula (III) and C1-C20 alkyl group of R⁸ and R⁹ in Formula (IV) have the same specific examples as the halogen atom and C1-C20 alkyl group of X exemplified above.
In addition, the substituent group of the C1-C20 alkyl group in R⁷ to R⁹ has the same examples as the substituent group of X. Preferred is a halogen atom.

Specific examples of the group represented by Formula (III) include ethynyl groups, 1-propynyl groups and 1-butynyl groups.
Specific examples of the group represented by Formula (IV) include 1-methoxyimino-ethyl and 1-ethoxyimino-ethyl groups.

The oxime ether derivative represented by Formula (I), or a salt thereof is preferably an oxime ether derivative represented by the following Formula (V) or (VI), or a salt thereof, more preferably, an oxime ether derivative represented by the following Formula (VI).

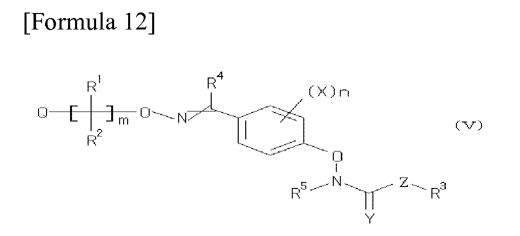

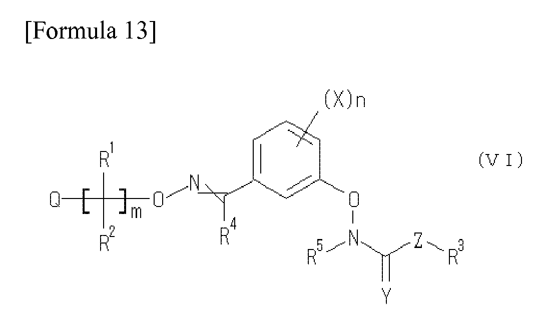

(wherein X, R¹ to R⁵, Y, Z, Q, m and n are defined as above.)

### (Specific examples of compound)

The compound of the present invention includes stereoisomers and mixtures thereof, based on carbon-nitrogen double bonds and asymmetric carbon atoms. In addition, the compound of the present invention also includes solvates or crystalline polymorphs, if present.
Any salt of the compound of the present invention is not particularly limited so long as it is an agriculturally acceptable salt. Examples of the salt include inorganic acids such as hydrochloric acid or sulfuric acid; organic acids such as acetic acid and lactic acid; and the like.

Examples of oxime ether derivatives of the present invention are set forth in Tables 1 to 28 below.
In the following Tables, Me represents a methyl group, Et represents an ethyl group, Ph represents a phenyl group, Np represents a naphthyl group, and c-Pr represents cyclopropyl.

[Table 1]

**TABLE 1**

| | | | | | | | |
|---|---|---|---|---|---|---|---|
| 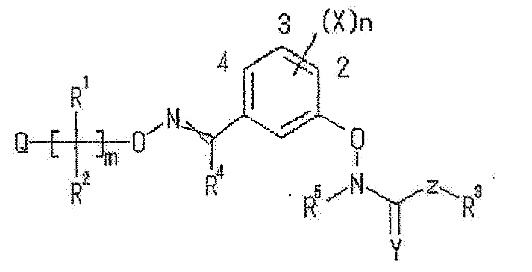 | | | | | | | |

| Compound No. | Q | [C(R¹)(R²)]m | R⁴ | R⁵ | (X)n | Y | Z-R³ |
|---|---|---|---|---|---|---|---|
| 1-1 | 2-CF₃-Ph | CH₂ | Me | H | 2-Cl | O | OMe |
| 1-2 | 3-CF₃-Ph | CH₂ | Me | H | 2-Cl | O | OMe |
| 1-3 | 4-CF₃-Ph | CH₂ | Me | H | 2-Cl | O | OMe |
| 1-4 | 2-F-Ph | CH₂ | Me | H | 2-Cl | O | OMe |
| 1-5 | 3-F-Ph | CH₂ | Me | H | 2-Cl | O | OMe |
| 1-6 | 4-F-Ph | CH₂ | Me | H | 2-Cl | O | OMe |
| 1-7 | 2-CF₃O-Ph | CH₂ | Me | H | 2-Cl | O | OMe |
| 1-8 | 3-CH₃O-PH | CH₂ | Me | H | 2-Cl | O | OMe |
| 1-9 | 2-F-3-CF₃-Ph | CH₂ | Me | H | 2-Cl | O | OMe |
| 1-10 | 2-F-4-CF₃-Ph | CH₂ | Me | H | 2-Cl | O | OMe |
| 1-11 | 2-F-5-CF₃-Ph | CH₂ | Me | H | 2-Cl | O | OMe |
| 1-12 | 2-F-6-CF3-Ph | CH₂ | Me | H | 2-Cl | O | OMe |
| 1-13 | 3-F-2-CF₃-Ph | CH₂ | Me | H | 2-Cl | O | OMe |
| 1-14 | 3-F-4-CF₃-Ph | CH₂ | Me | H | 2-Cl | O | OMe |
| 1-15 | 3-F-5-CF₃-Ph | CH₂ | Me | H | 2-Cl | O | OMe |
| 1-16 | 3-F-6-CF₃-Ph | CH₂ | Me | H | 2-Cl | O | OMe |
| 1-17 | 4-P-2-CF₃-Ph | CH₂ | Me | H | 2-Cl | O | OMe |
| 1-18 | 4-F-3-CF₃-Ph | CH₂ | Me | H | 2-Cl | O | OMe |
| 1-19 | 4-F-5-CF₃-Ph | CH₂ | Me | H | 2-Cl | O | OMe |
| 1-20 | 4-F-6-CF₃-Ph | CH₂ | Me | H | 2-Cl | O | OMe |

[Table 2]

**TABLE 2**

| | | | | | | | |
|---|---|---|---|---|---|---|---|
| 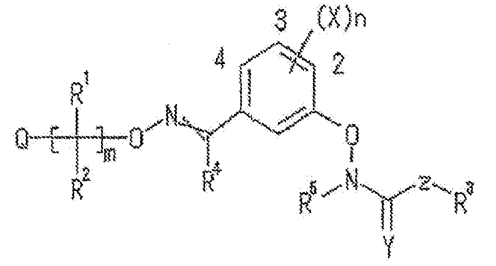 | | | | | | | |

| Compound No. | Q | [C(R¹)(R²)]m | R⁴ | R⁵ | (X)n | Y | Z-R³ |
|---|---|---|---|---|---|---|---|
| 2-1 | 2,3-F₂-Ph | CH₂ | Me | H | 2-Cl | O | OMe |
| 2-2 | 2,4-F₂-Ph | CH₂ | Me | H | 2-Cl | O | OMe |
| 2-3 | 3,4-F₂-Ph | CH₂ | Me | H | 2-Cl | O | OMe |
| 2-4 | 3,5-F₂-Ph | CH₂ | Me | H | 2-Cl | O | OMe |
| 2-5 | 2,6-F₂-Ph | CH₂ | Me | H | 2-Cl | O | OMe |
| 2-6 | 2,3,4-F₃-Ph | CH₂ | Me | H | 2-Cl | O | OMe, |
| 2-7 | 2-(FCH₂CH₂)-Ph | CH₂ | Me | H | 2-Cl | O | OMe |
| 2-8 | 3-(FCH₂CH₂)-Ph | CH₂ | Me | H | 2-Cl | O | OMe |
| 2-9 | 4-(FCH₂CH₂)-Ph | CH₂ | Me | H | 2-Cl | O | OMe |
| 2-10 | 2,4-(CF₃O)₂-Ph | CH₂ | Me | H | 2-Cl | O | OMe |
| 2-11 | 2,3+(CF₃O)₂-Ph | CH₂ | Me | H | 2-Cl | O | OMe |
| 2-12 | 3-CF₃-1-Np | CH₂ | Me | H | 2-Cl | O | OMe |
| 2-13 | 4-CF₃-1-Np | CH₂ | Me | H | 2-Cl | O | OMe |
| 2-14 | 3,4-(CF₃)₂-1-Np | CH₂ | Me | H | 2-Cl | O | OMe |
| 2-15 | 2-F-1-Np | CH₂ | Me | H | 2-Cl | O | OMe |
| 2-16 | 3-F-1-Np | CH₂ | Me | H | 2-Cl | O | OMe |
| 2-17 | 2,2-F₂-Np | CH₂ | Me | H | 2-Cl | O | OMe |
| 2-18 | 2,3,4-F₃-1-Np. | CH₂ | Me | H | 2-Cl | O | OMe |
| 2-19 | 2-Me-3-CF₃-Ph | CH₂ | Me | H | 2-Cl | O | OMe |

[Table 3]

**TABLE 2-(continued)**

| Compound No. | Q | [C(R1)(R²)]m | R⁴ | R⁵ | (X)n | Y | Z-R³ |
|---|---|---|---|---|---|---|---|
| 2-20 | 2-Me4-CF3-Ph | CH₂ | Me | H | 2-Cl | O | OMe |
| 2-21 | 2-Me-5-CF₃-Ph | CH₂ | Me | H | 2-Cl | O | OMe |
| 2-22 | 2-Me-6-CF₃-Ph | CH₂ | Me | H | 2-Cl | O | OMe |
| 2-23 | 3-Me-2-CF₃-Ph | CH₂ | Me | H | 2-Cl | O | OMe |
| 2-24 | 3-Me-4-CF₃-Ph | CH₂ | Me | H | 2-Cl | O | OMe |
| 2-25 | 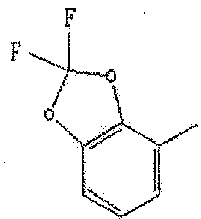 | CH₂ | Me | H | 2-Cl | O | OMe |

[Table 4]

**TABLE 3**

| | | | | | | | |
|---|---|---|---|---|---|---|---|
| 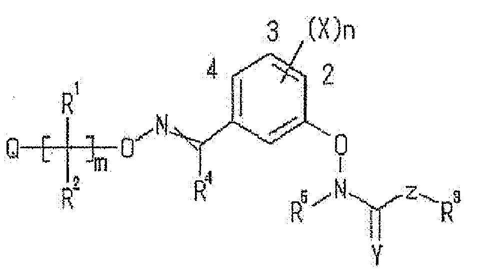 | | | | | | | |

| Compound No. | Q | [C(R¹)(R²)]m | R⁴ | R⁵ | (X)n | Y | Z-R³ |
|---|---|---|---|---|---|---|---|
| 3-1 | 2-CF₃-Ph | CH₂ | Me | CHO | 2-Cl | O | OMe |
| 3-2 | 3-CF₃-Ph | CH₂ | Me | CHO | 2-Cl | O | OMe |
| 3-3 | 4-CF₃-Ph | CH₂ | Me | CHO | 2-Cl | O | OMe |
| 3-4 | 2-F-Ph | CH₂ | Me | CHO | 2-Cl | O | OMe |
| 3-5 | 3-F-Ph | CH₂ | Me | CHO | 2-Cl | O | OMe |
| 3-6 | 4-F-Ph | CH₂ | Me | CHO | 2-Cl | O | OMe |
| 3-7 | 2-CF₃O-Ph | CH₂ | Me | CHO | 2-Cl | O | OMe |
| 3-8 | 3-CF₃O-ph | CH₂ | Me | CHO | 2-Cl | O | OMe |
| 3-9 | 2-F-3-CF₃-Ph | CH₂ | Me | CHO | 2-Cl | O | OMe |
| 3-10 | 2-F-4-CF₃-Ph | CH₂ | Me | CHO | 2-Cl | O | OMe |
| 3-11 | 2-F-5-CF₃-Ph | CH₂ | Me | CHO | 2-Cl | O | OMe |
| 3-12 | 2-F-6-CF₃-Ph | CH₂ | Me | CHO | 2-Cl | O | OMe |
| 3-13 | 3-F-2-CF₃-Ph | CH₂ | Me | CHO | 2-Cl | O | OMe |
| 3-14 | 3-F-4-CF₃-Ph | CH₂ | Me | CHO | 2.Cl | O | OMe |
| 3-15 | 3-F-5-CF₃-Ph | CH₂ | Me | CHO | 2-Cl | O | OMe |
| 3-16 | 3-F-6-CF₃-Ph | CH₂ | Me | CHO | 2-Cl | O | OMe |
| 3-17 | 4-F-2-CF₃-Ph | CH₂ | Me | CEO | 2-Cl | O | OMe |
| 3-18 | 4-F-3-CF₃-Ph | CH₂ | Me | CHO | 2-Cl | O | OMe |
| 3-19 | 4-F-5-CF₃-Ph | CH₂ | Me | CHO | 2-Cl | O | OMe |
| 3-20 | 4-F-6-CF₃-Ph | CH₂ | Me | CHO | 2-Cl | O | OMe |

[Table 5]

**TABLE 4**

| | | | | | | | |
|---|---|---|---|---|---|---|---|
| 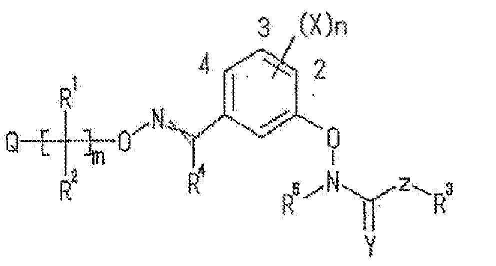 | | | | | | | |

| Compound No | Q | [C(R¹)(R²)]m | R⁴ | R⁵ | (X)n | Y | Z-R³ |
|---|---|---|---|---|---|---|---|
| 4-1 | 2-CF₃-Ph | CH₂ | Me | H | 2-F | O | OMe |
| 4-2 | 3-CF₃-Ph | CH₂ | Me | H | 2-F | O | OMe |
| 4-3 | 4-CF₃-Ph | CH₂ | Me | H | 2-F | O | OMs |
| 4-4 | 2-F-Ph | CH₂ | Me | H | 2-F | O | OMe |
| 4-5 | 3-F-Ph | CH₂ | Me | H | 2-F | O | OMe |
| 4-6 | 4-F-Ph | CH₂ | Me | H | 2-F | O | OMe |
| 4-7 | 2-CF₃O-Ph | CH₂ | Me | H | 2-F | O | OMe |
| 4-8 | 3-CF₃O-Ph | CH₂ | Me | H | 2-F | O | OMe |
| 4-9 | 2-F-3-CF₃-Ph | CH₂ | Me | H | 2-F | O | OMe |
| 4-10 | 2-F-4-CF₃-Ph | CH₂ | Me | H | 2-F | O | OMe |
| 4-11 | 2-F-5-CF₃-Ph | CH₂ | Me | H | 2-F | O | OMe |
| 4-12 | 2-F-6-CF₃-Ph | CH₂ | Me | H | 2-F | O | OMe |
| 4-13 | 3-F-2-CF₃-Ph | CH₂ | Me | H | 2-F | O | OMe |
| 4-14 | 3-F-4-CF₃-Ph | CH₂ | Me | H | 2-F | O | OMe |
| 4-15 | 3-F-5-CF₃-Ph | CH₂ | Me | H | 2-F | O | OMe |
| 4-16 | 3-F-6-CF₃-Ph | CH₂ | Me | H | 2-F | O | OMe |
| 4-17 | 4-F-2-CF₃-Ph | CH₂ | Me | H | 2-F | O | OMe |
| 4-18 | 4-F-3-CF₃-Ph | CH₂ | Me | H | 2-F | O | OMe |
| 4-19 | 4-F-5-CF₃-Ph | CH₂ | Me | H | 2-F | O | OMe |
| 4-20 | 4-F-6-CF₃-Ph | CH₂ | Me | H | 2-F | O | OMe |

[Table 6]

**TABLE 5**

| | | | | | | | |
|---|---|---|---|---|---|---|---|
| 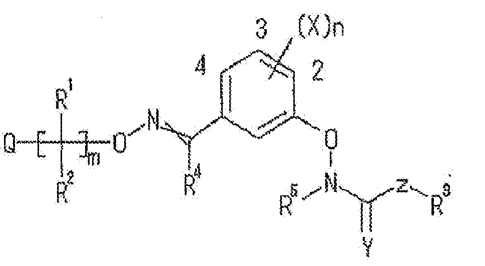 | | | | | | | |

| Compound No. | Q | [C(R¹)(R²)]m | R⁴ | R⁵ | (X)n | Y | Z-R³ |
|---|---|---|---|---|---|---|---|
| 5-1 | 2-CF₃-Ph | (CH₂)₂ | Me | H | 2-Cl | O | OMe |
| 5-2 | 3-CF₃-Ph | (CH₂)₂ | Me | H | 2-Cl | O | OMe |
| 5.3 | 4-CF₃-Ph | (CH₂)₂ | Me | H | 2-Cl | O | OMe |
| 5-4 | 2-F-Ph | (CH₂)₂ | Me | H | 2-Cl | O | OMe |
| 5-3 | 3-F-Ph | (CH₂)₂ | Me | H | 2-Cl | O | OMe |
| 5-6 | 4-F-Ph | (CH₂)₂ | Me | H | 2-Cl | O | OMe |
| 5-7 | 2-CF₃O-Ph | (CH₂)₂ | Me | H | 2-Cl | O | OMe |
| 5-8 | 3-CF₃O-Ph | (CH₂)₂ | Me | H | 2-Cl | O | OMe |
| 5-9 | 2-F-3-CF₃-Ph | (CH₂)₂ | Me | H | 2-Cl | O | OMe |
| 5-10 | 2-F-4-CF₃-Ph | (CH₂)₂ | .Me | H | 2-Cl | O | OMe |
| 5-11 | 2-F-5-CF₃-Ph | (CH₂)₂ | Me | H | 2-Cl | O | OMe |
| 5-12 | 2-F-6-CF₃-Ph | (CH₂)₂ | Me | H | 2-Cl | O | OMe |
| 5-13 | 3-F-2-CF₃-Ph | (CH₂)₂ | Me | H | 2-Cl | O | OMe |
| 5-14 | 3-F-4-CF₃-Ph | (CH₂)₂ | Me | H | 2-Cl | O | OMe |
| 5-15 | 3-F-5-CF₃-Ph | (CH₂)₂ | Me | H | 2-Cl | O | OMe |
| 5-16 | 3-F-6-CF₃-Ph | (CH₂)₂ | Me | H | 2-Cl | O | OMe |
| 5-17 | 4-F-2-CF₃-Ph | (CH₂)₂ | Me | H | 2-Cl | O | OMe |
| 5-18 | 4-F-3-CF₃-Ph | (CH₂)₂ | Me | H | 2-Cl | O | OMe |
| 5-19 | 4-F-5-CF₃-Ph | (CH₂)₂ | Me | H | 2-Cl | O | OMe |
| 5-20 | 4-F-6-CF₃-Ph | (CH₂)₂ | Me | H | 2-Cl | O | OMe |

[Table 7]

**TABLE 6**

| | | | | | | | |
|---|---|---|---|---|---|---|---|
| 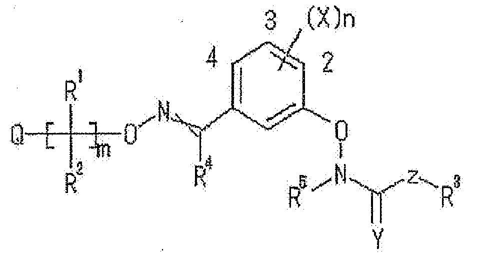 | | | | | | | |

| Compound No. | Q | [C(R¹)(R²)]m | R⁴ | R⁵ | (X)n | Y | Z-R³ |
|---|---|---|---|---|---|---|---|
| 6-1 | 2-CF₃-Ph | CH₂ | Me | H | 2-Cl | O | OCH=CH₂ |
| 6-2 | 3-CF₃-Ph | CH₂ | Me | H | 2-Cl | O | OCH=CH₂ |
| 6-3 | 4-CF₃-Ph | CH₂ | Me | H | 2-Cl | O | OCH=CH₂ |
| 6-4 | 2-F-Ph | CH₂ | Me | H | 2-Cl | O | OCH=CH₂ |
| 6-5 | 3-F-Ph | CH₂ | Me | H | 2-Cl | O | OCH=CH₂ |
| 6-6 | 4-F-Ph | CH₂ | Me | H | 2-Cl | O | OCH=CH₂ |
| 6-7 | 2-CF₃O-Ph | CH₂ | Me | H | 2-Cl | O | OCH=CH₂ |
| 6-8 | 3-CF₃O-Ph | CH₂ | Me | H | 2-Cl | O | OCH=CH₂ |
| 6-9 | 2-F-3-CF₃-Ph | CH₂ | Me | H | 2-Cl | O | OCH=CH₂ |
| 6-10 | 2-P-4-CF₃-Ph | CH₂ | Me | H | 2-Cl | O | OCH=CH₂ |
| 6-11 | 2-F-5-CF₃-Ph | CH₂ | Me | H | 2-Cl | O | OCH=CH₂ |
| 6-12 | 2-F-6-CF₃-Ph | CH₂ | Me | H | 2-Cl | O | OCH=CH₂ |
| 6-13 | 3-F-2-CF₃-Ph | CH₂ | Me | H | 2-Cl | O | OCH=CH₂ |
| 6-14 | 3-F-4-CF₃-Ph | CH₂ | Me | H | 2-Cl | O | OCH=CH₂ |
| 6-15 | 3-F-5-CF₃-Ph | CH₂ | Me | H | 2-Cl | O | OCH=CH₂ |
| 6-16 | 3-F-6-CF₃-Ph | CH₂ | Me | H | 2-Cl | O | OCH=CH₂ |
| 6-17 | 4-F-2-CF₃-Ph | CH₂ | Me | H | 2-Cl | O | OCH=CH₂ |
| 6-18 | 4-F-3-CF₃-Ph | CH₂ | Me | H | 2-Cl | O | OCH=CH₂ |
| 6-19 | 4-F-5-CF₃-Ph | CH₂ | Me | H | 2-Cl | O | OCH=CH₂ |
| 6-20 | 4-F-6-CF₃-Ph | CH₂ | Me | H | 2-Cl | O | OCH=CH₂ |

[Table 8]

**TABLE 7**

| | | | | | | | |
|---|---|---|---|---|---|---|---|
| 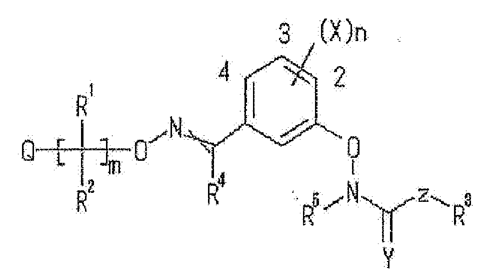 | | | | | | | |

| Compound No. | Q | [C(R¹)(R²)]ₘ | R⁴ | R⁵ | (X)n | Y | Z-R³ |
|---|---|---|---|---|---|---|---|
| 7-1 | 2-CF₃-Ph | [CH(CH₃)] | Me | H | 2-Cl | O | OMe |
| 7-2 | 3-CF₃-Ph | [CH(CH₃)] | Me | H | 2-Cl | O | OMe |
| 7-3 | 4-CF₃-Ph | [CH(CH₃)] | Me | H | 2-Cl | O | OMe |
| 7-4 | 2-F-Ph | [CH(CH₃)] | Me | H | 2-Cl | O | OMe |
| 7-5 | 3-F-Ph | [CH(CH₃)] | Me | H | 2-Cl | O | OMe |
| 7-6 | 4-F-Ph | [CH(CH₃)] | Me | H | 2-Cl | O | OMe |
| 7-7 | 2-CF₃O-Ph | [CH(CH₃)] | Me | H | 2-Cl | O | OMe |
| 7-8 | 3-CF₃O-Ph | [CH(CH₃)] | Me | H | 2-Cl | O | OMe |
| 7-9 | 2-F-3-CF₃-Ph | [CH(CH₃)] | Me | H | 2-Cl | O | OMe |
| 7-10 | 2-F4-CF₃-Ph | [CH(CH₃)] | Me | H | 2-Cl | O | OMe |
| 7-11 | 2-CF₃-Ph | (CH₂)₄ | Me | H | 2-Cl | O | OMe |
| 7-12 | 3-CF₃-Ph | (CH₂)₄ | Me | H | 2-Cl | O | OMe |
| 7-13 | 4-CF₃-Ph | (CH₂)₄ | Me | H | 2-Cl | O | OMe |
| 7-14 | 2-F-Ph | (CH₂)₄ | Me | H | 2-Cl | O | OMe |
| 7-15 | 3-F-Ph | (CH₂)₄ | Me | H | 2-Cl | O | OMe |

[Table 9]

**TABLE 7-(continued)**

| Compound No. | Q | [C(R¹)(R²)]m | R⁴ | R⁵ | (X)n | Y | Z-R³ |
|---|---|---|---|---|---|---|---|
| 7-16 | 2-CF₃-Ph | 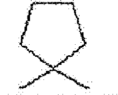 | Me | H | 2-Cl | O | OMe |
| 7-17 | 2-F-3-CF₃O-Ph | 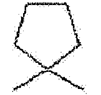 | Me | H | 2-Cl | O | OMe |
| 7-18 | 2-CF₃-Ph | 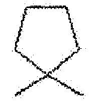 | Me | H | 2-Cl | O | OMe |
| 7-19 | 2-F-3-CF₃O-Ph | 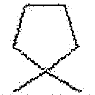 | Me | H | 2-Cl | O | OMe |
| 7-20 | 3-CF₃O-Ph | 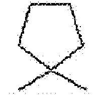 | Me | H | 2-Cl | O | OMe |

[Table 10]

**TABLE 8**

| | | | | | | | |
|---|---|---|---|---|---|---|---|
| 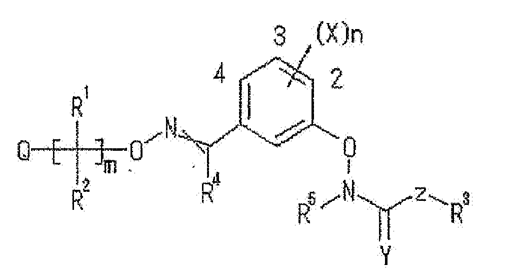 | | | | | | | |

| Compound No. | Q | [C(R¹)(R²)]m | R⁴ | R⁵ | (X)n | Y | Z-R³ |
|---|---|---|---|---|---|---|---|
| 8-1 | 2-CF₃-Ph | CH₂ | Me | H | 2-Cl | S | OMe |
| 8-2 | 3-CF₃-Ph | CH₂ | Me | H | 2-Cl | S | OMe |
| 8-3 | 4-CF₃-Ph | CH₂ | Me | H | 2-Cl | S | OMe |
| 8-4 | 2-F-Ph | CH₂ | Me | H | 2-Cl | S | OMe |
| 8-5 | 3-F-Ph | CH₂ | Me | H | 2-Cl | S | OMe |
| 8-6 | 4-F-Ph | CH₂ | Me | H | 2-Cl | S | OMe |
| 8-7 | 2-CF₃O-Ph | CH₂ | Me | H | 2-Cl | S | OMe |
| 8-8 | 3-CF₃O-Ph | CH₂ | Me | H | 2-Cl | S | OMe |
| 8-9 | 2-CF₃C(=O)-Ph | CH₂ | Me | H | 2-Cl | S | OMe |
| 8-10 | 3-CP₃C(=O)-Ph | CH₂ | Me | H | 2-Cl | O | OMe |
| 8-11 | 4-CF₃C(=O)-Ph | CH₂ | Me | H | 2-Cl | O | OMe |
| 8-12 | 3-(2-F-EtO)-Ph | CH₂ | Me | H | 2-Cl | O | OMe |
| 8-13 | 4-(2-F-EtO)-Ph | CH₂ | Me | H | 2-Cl | O | OMe |
| 8-14 | 3,4-(CF₃O)₂-Ph | CH₂ | Me | H | 2-Cl | O | OMe |
| 8-15 | 2,4-(CF₃O)₂-Ph | CH₂ | Me | H | 2-Cl | O | OMe |
| 8-16 | 3-CF₃-Ph | CH₂ | Me | H | 2-F | O | OEt |
| 8-17 | 4-CF₃-Ph | CH₂ | Me | H | 2-F | O | OEt |
| 8-18 | 2-F-Ph | CH₂ | Me | H | 2-F | O | OEt |
| 8-19 | 3-F-Ph | CH₂ | Me | H | 2-F | O | OEt |
| 8-20 | 3-CF₃O-Ph | CH₂ | Me | H | 2-F | O | OEt |

[Table 11]

**TABLE 9**

| | | | | | | | |
|---|---|---|---|---|---|---|---|
| 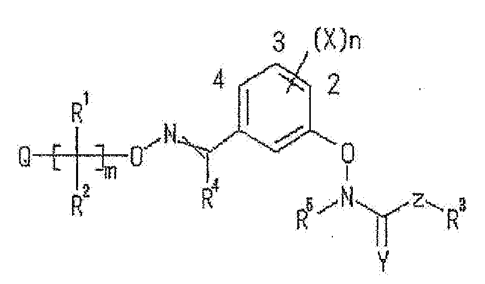 | | | | | | | |

| Compound No. | Q | [C(R¹)(R²)]m | R⁴ | R⁵ | (X)n | Y | Z-R³ |
|---|---|---|---|---|---|---|---|
| 9-1 | 2-CF₃-Ph | CH₂ | CHF₂ | H | 2-Cl | O | OMe |
| 9-2 | 2-CF₃-Ph | CH₂ | CF₃ | H | 2-Cl | O | OMe |
| 9-3 | 2-CF₃-Ph | CH₂ | H | H | 2-Cl | O | OMe |
| 9-4 | 2-CF₃-Ph | CH₂ | C≡N | H | 2-Cl | O | OMe |
| 9-5 | 4-F-2-CF₃-Ph | CH₂ | C≡N | H | 2-Cl | O | OMe |
| 9-6 | 2-CF₃-Ph | CH₂ | NH₂ | H | 2-Cl | O | OMe |
| 9-7 | 2-CF₃-Ph | CH₂ | Me | H | 2-CHF₂ | O | OMe |
| 9-8 | 2-CF₃-Ph | CH₂ | Me | H | 2-Cl-3-F | O | OMe |
| 9-9 | 2-CF₃C(=O)-Ph | CH₂ | CHF₂ | H | 2-Cl | S | OMe |
| 9-10 | 3-CF₃C(=O)-Ph | CH₂ | CF₃ | H | 2-Cl | O | OMe |
| 9-11 | 2-CF₃-Ph | CH₂ | Me | H | 2-Cl | O | OtBu |
| 9-12 | 2-CF₃-Ph | CH₂ | Me | H | 2-Cl | O | OEt |
| 9-13 | 3-CF₃-Ph | [CH(C₂H₅))] | Me | H | 2-Cl | O | OMe |
| 9-14 | 2-CF₃-Ph | CH₂ | Me | AcOCH₂ | 2-Cl | O | OMe |
| 9-15 | 1-Ethoxyimino- ethyl | [CH(CH₃)] | Me | H | 2-Cl | O | One |
| 9-16 | tBu | - | Me | H | 2-Cl | O | OMe |

[Table 12]

**TABLE 10**

| | | | | | | | |
|---|---|---|---|---|---|---|---|
| 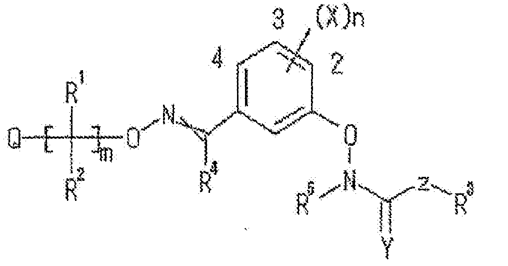 | | | | | | | |

| Compound | Q | [C(R¹)(R²)]m | R⁴ | R⁵ | (X)n | Y | Z-R³ |
|---|---|---|---|---|---|---|---|
| 11-1 | Ph | -- | Me | H | 2-Cl | O | OMe |
| 11-2 | Ph | CH₂ | Me | H | 2-Cl | O | OMe |
| 11-3 | 2-Cl-Ph | CH₂ | Me | H | 2-Cl | O | OMe |
| 114 | 3-Cl-Ph | CH₂ | Me | H | 2-Cl | O | OMe |
| 11-5 | 4-Cl-Ph | CH₂ | Me | H | 2-Cl | O | OMe |
| 11-6 | 2-Br-Ph | CH₂ | Me | H | 2-Cl | O | OMe |
| 11-7 | 3-Br-Ph | CH₂ | Me | H | 2-Cl | O | OMe |
| 11-8 | 4-Br-Ph | CH₂ | Me | H | 2-Cl | O | OMe |
| 11-9 | 2-I-Ph | CH₂ | Me | H | 2-Cl | O | OMe |
| 11-10 | 3-I-Ph | CH₂ | Me | H | 2-Cl | O | OMe |
| 11-11 | 4-I-Ph | CH₂ | Me | He | 2-Cl | O | OMe |
| 11-12 | 2-MeO-Ph | CH₂ | Me | H | 2-Cl | O | OMe |
| 11-13 | 3-MeO-Ph | CH₂ | Me | H | 2-Cl | O | OMe |
| 11-14 | 4-MeO-Ph | CH₂ | Me | H | 2-Cl | O | OMe |
| 11-15 | 2-MeS-Ph | CH₂ | Me | H | 2-Cl | O | OME |
| 11-16 | 2-MeS(=O)-Ph | CH₂ | Me | H | 2-Cl | O | OMe |
| 11-17 | 2-MeS(=O)₂-Ph | CH₂ | Me | H | 2-Cl | O | OMe |
| 11-18 | 3-MeS-Ph | CH₂ | Me | H | 2-Cl | O | OMe |
| 11-19 | 3-MsS(=O)-Ph | CH₂ | Me | H | 2-Cl | O | OMe |
| 11-20 | 3-MeS(=O)₂-Ph | CH₂ | Me | H | 2-Cl | O | OMe |

[Table 13]

**TABLE 10-(continued)**

| Compound No. | Q | [C(R¹)(R²)]m | R⁴ | R⁵ | (X)n | Y | Z-R³ |
|---|---|---|---|---|---|---|---|
| 11-21 | 2-NO₂-Ph | CH₂ | Me | H | 2-Cl | O | OMe |
| 11-22 | 2-N≡C-Ph | CH₂ | Me | H | 2-Cl | O | OMe |
| 11-23 | 2-MeOC(=O)-Ph | CH₂ | Me | H | 2-Cl | O | OMe |
| 11-24 | 3-MeOC(=O)-Ph | CH₂ | Me | H | 2-Cl | O | OMe |
| 11-25 | H-C≡C | CH₂ | Me | H | 2-Cl | O | OMe |
| 11-26 | Me-C≡C | CH₂ | Me | H | 2-Cl | O | OMe |
| 11-27 | MeOC(=O)C(=NOMe)-Ph | CH₂ | Me | H | 2-Cl | O | OMe |

[Table 14]

**TABLE 11**

| | | | | | | | |
|---|---|---|---|---|---|---|---|
| 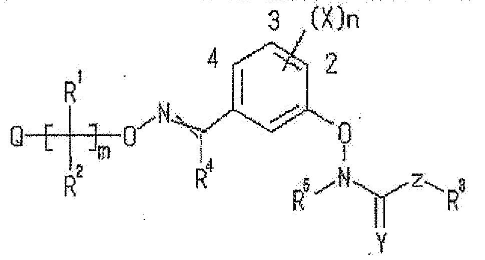 | | | | | | | |

| Compound No. | Q | [C(R¹)(R²)]m | R⁴ | R⁵ | (X)n | Y | Z-R³ |
|---|---|---|---|---|---|---|---|
| 12-1 | 2,3-Cl₂-Ph | CH₂ | Me | H | 2-Cl | O | OMe |
| 12-2 | 2,4-Cl₂-Ph | CH₂ | Me | H | 2-Cl | O | OMe |
| 12-3 | 3,4-Cl₂-Ph | CH₂ | Me | H | 2-Cl | O | OMe |
| 12-4 | 3,5-Cl₂-Ph | CH₂ | Me | H | 2-Cl | O | OMe |
| 12-5 | 2,6-Cl₂-Ph | CH₂ | Me | H | 2-Cl | O | OMe |
| 12-6 | 2,3,4-Cl₃-Ph | CH₂ | Me | H | 2-Cl | O | OMe |
| 12-7 | 2-(ClCH₂CH₂)-Ph | CH₂ | Me | H | 2-Cl | O | OMe |
| 12-8 | 2-EtO-Ph | CH₂ | Me | H | 2-Cl | O | OMe |
| 12-9 | 3-EtO-Ph | CH₂ | Me | H | 2-Cl | O | OMe |
| 12-10 | 4-EtO-Ph | CH₂ | Me | H | 2-Cl | O | OMe |
| 12-11 | 2,3-(MeO)₂-Ph | CH₂ | Me | H | 2-Cl | O | OMe |
| 12-12 | 2,4-(MeO)₂-Ph | CH₂ | Me | H | 2-Cl | O | OMe |
| 12-13 | 2,4-(EtO)₂-Ph | CH₂ | Me | H | 2-Cl | O | OMe |
| 12-14 | 3,4-Me₂-1-Np | CH₂ | Me | H | 2-Cl | O | OMe |
| 12-15 | Np | CH₂ | Me | H | 2-Cl | O | OMe |
| 12-16 | 3-Me-1-Np | CH₂ | Me | H | 2-Cl | O | OMe |
| 12-17 | 2,3-EtS-Ph | CH₂ | Me | H | 2-Cl | O | OMe |
| 12-18 | 2,4-EtS-Ph | CH₂ | Me | H | 2-Cl | O | OMe |
| 12-19 | 2-Me-3-MeS-Ph | CH₂ | Me | H | 2-Cl | O | OMe |
| 12-20 | 2-Me-4-MeS-Ph | CH₂ | Me | H | 2-Cl | O | OMe |

[Table 15]

**TABLE 11-(continued)**

| compound No. | Q | [C(R²)(R²)]m | R⁴ | R⁵ | (X)n | Y | Z-R³ |
|---|---|---|---|---|---|---|---|
| 12-21 | 2-Me-5-MeS-Ph | CH₂ | Me | H | 2-Cl | O | OMe |
| 12-22 | 2-Me-6-MeS-Ph | CH₂ | Me | H | 2-Cl | O | OMe |
| 12-23 | 2-Ets-4-Me-Ph | CH₂ | Me | H | 2-Cl | O | OMe |
| 12-24 | 2-Et-5-MeS-Ph | CH₂ | Me | H | 2-Cl | O | OMe |
| 12-25 | 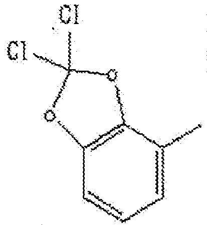 | CH₂ | Me | H | 2-Cl | O | OMe |

[Table 16]

**TABLE 12**

| | | | | | | | |
|---|---|---|---|---|---|---|---|
| 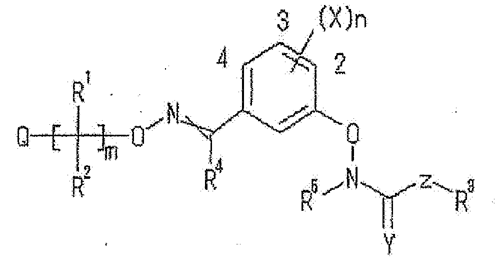 | | | | | | | |

| Compound No. | Q | [C(R¹)(R²)m | R⁴ | R⁵ | (X)n | Y | Z-R³ |
|---|---|---|---|---|---|---|---|
| 13-1 | Ph | - | Me | CHO | 2-Cl | O | OMe |
| 13-2 | Ph | CH₂ | Me | CHO | 2-Cl | O | OMe |
| 13-3 | 2-Cl-Ph | CH₂ | Me | CHO | 2-Cl | O | OMe |
| 13-4 | 3-Cl-Ph | CH₂ | Me | CHO | 2-Cl | O | OMe |
| 13-5 | 4-Cl-Ph | CH₂ | Me | CHO | 2-Cl | O | OMe |
| 13-6 | 2-Br-Ph | CH₂ | Me | CHO | 2-Cl | O | OMe |
| 13-7 | 3-Br-Ph | CH₂ | Me | CHO | 2-Cl | O | OMe |
| 13-8 | 4-Br-Ph | CH₂ | Me | CHO | 2-Cl | O | OMe |
| 13-9. | 2-1-Ph | CH₂ | Me | CHO | 2-Cl | O | OMe |
| 13-10 | 2-MeO-Ph | CH₂ | Me | CHO | 2-Cl | O | OMe |
| 13-11 | 3-MeO-Ph | CH₂ | Me | CHO | 2-Cl | O | OMe |
| 13-12 | 4-MeO-Ph | CH₂ | Me | CHO | 2-Cl | O | OMe |
| 13-13 | 2-MeS-Ph | CH₂ | Me | CHO | 2-Cl | O | OMe |
| 13-14 | 2-MeS(=O)-Ph | CH₂ | Me | CHO | 2-Cl | O | OMe |
| 13-15 | 2-MeS(=O)₂-Ph | CH₂ | Me | CHO | 2-Cl | O | OMe |
| 13-16 | 3-MeS-Ph | CH₂ | Me | CHO | 2-Cl | O | OMe |
| 13-17 | 3-MeS(=O)-Ph | CH₂ | Me | CHO | 2-Cl | O | OMe |
| 13-18 | 3-MeS(=O)₂-Ph | CH₂ | Me | CHO | 2-Cl | O | OMe |
| 13-19 | H-C≡C | CH₂ | Me | CHO | 2-Cl | O | OMe |
| 13-20 | Me-C≡C | CH₂ | Me | CHO | 2-Cl | O | OMe |

[Table 17]

**TABLE 13**

| | | | | | | | |
|---|---|---|---|---|---|---|---|
| 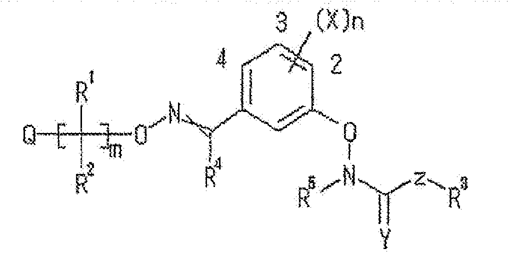 | | | | | | | |

| Compound | Q | [C(R²)(R²)]m | R⁴ | R⁵ | (X)n | Y | Z-R³ |
|---|---|---|---|---|---|---|---|
| 14-1 | Ph | CH₂ | Me | H | 2-F | O | OMe |
| 14-2 | Ph | CH₂ | Me | H | 2-F | O | OMe |
| 14-3 | 2-Cl-Ph | CH₂ | Me | H | 2-F | O | OMe |
| 14-4 | 3-Cl-Ph | CH₂ | Me | H | 2-F | O | OMe |
| 14-5 | 4-Cl-Ph | CH₂ | Me | H | 2-F | O | OMe |
| 14-6 | 2-Br-Ph | CH₂ | Me | H | 2-F | O | OMe |
| 14-7 | 3-Br-Ph | GH₂ | Me | H | 2-F | O | OMe |
| 14-8 | 4-Br-Ph | CH₂ | Me | H | 2-F | O | OMe |
| 14-19 | 2-I-Ph | CH₂ | Me | H | 2-F | O | OMe |
| 14-10 | 2-MeO-Ph | CH₂ | Me | H | 2-F | O | OMe |
| 14-11 | 3-MeO-Ph | CH₂ | Me | H | 2-F | O | OMe |
| 14-12 | 4-MeO-Ph | CH₂ | Me | H | 2-F | O | OMe |
| 14-13 | 2-MeS-Ph | CH₂ | Me | H | 2-F | O | OMe |
| 14-14 | 2-MeS(=O)-Ph | CH₂ | Me | H | 2-F | O | OMe |
| 14-15 | 2-MeS(=O)₂-Ph | CH₂ | Me | H | 2-F | O | OMe |
| 14-16 | 3-MeS-Ph | CH₂ | Me | H | 2-F | O | OMe |
| 14-17 | 3-MeS(=O)-Ph | CH₂ | Me | H | 2-F | O | OMe |
| 14-18 | 3-MeS(=O)₂-Ph | CH₂ | Me | H | 2-F | O | OMe |
| 14-19 | H-C≡C | CH₂ | Me | H | 2-F | O | OMe |
| 14-20 | Me-C≡C | CH₂ | Me | H | 2-F | O | OMe |

[Table 18]

**TABLE 14**

| | | | | | | | |
|---|---|---|---|---|---|---|---|
| 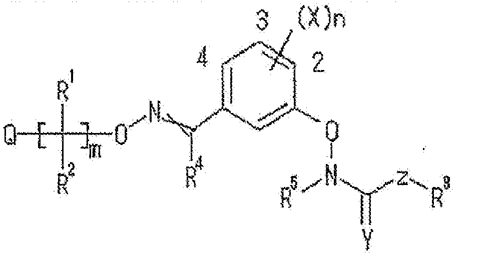 | | | | | | | |

| Compound No | Q | [C(R¹)(R²]m | R⁴ | R⁵ | (X)n | Y | Z-R³ |
|---|---|---|---|---|---|---|---|
| 15-1 | Ph | (CH₂)₂ | Me | H | 2-Cl | O | OMe |
| 15-2 | 2-Cl-Ph | (CH₂)₂ | Me | H | 2-Cl | O | OMe |
| 15-3 | 3-Cl-Ph | (CH₂)₂ | Me | H | 2-Cl | O | OMe |
| 15-4 | 4-Cl-Ph | (CH₂)₂ | Me | H | 2-Cl | O | OMe |
| 15-5 | 2-Br-Ph | (CH₂)₂ | Me | H | 2-Cl | O | OMe |
| 15-6 | 3-Br-Ph | (CH₂)₂ | Me | H | 2-Cl | O | OMe |
| 15-7 | 4-Br-Ph | (CH₂)₂ | Me | H | 2-Cl | O | OMe |
| 15-8 | 2-I-Ph | (CH₂)2 | Me | H | 2-Cl | O | OMe |
| 15-9 | 2-MeO-Ph | (CH₂)₂ | Me | H | 2-Cl | O | OMe |
| 15-10 | 3-MeO-Ph | (CH₂)₂ | Me | H | 2-Cl | O | OMe |
| 15-11 | 4-MeO-Ph | (CH₂)₂ | Me | H | 2-Cl | O | OMe |
| 15-12 | 2-MeS-Ph | (CH₂)₂ | Me | H | 2-Cl | O | OMe |
| 15-13 | 2-MeS(=O)-Ph | (CH₂)₂ | Me | H | 2-Cl | O | OMe |
| 15-14 | 2-MeS(=O)₂-Ph | (CH₂)₂ | Me | H | 2-Cl | O | OMe |
| 15-15 | 3-MeS-Ph | (CH₂)₂ | Me | H | 2-Cl | O | OMe |
| 15-16 | 3-MeS(=O)-Ph | (CH₂)₂ | Me | H | 2-Cl | O | OMe |
| 15-17 | 3-MeS(=O)₂-Ph | (CH₂)₂ | Me | H | 2-Cl | O | OMe |
| 15-18 | H-C≡C | (CH₂)₂ | Me | H | 2-Cl | O | OMe |
| 15-19 | Me-C≡C | (CR₂)₂ | Me | H | 2-Cl | O | OMe |
| 15-20 | n-Bu-C≡C | (CH₂)₂ | Me | H | 2-Cl | O | OMe |

[Table 19]

**TABLE 15**

| | | | | | | | |
|---|---|---|---|---|---|---|---|
| 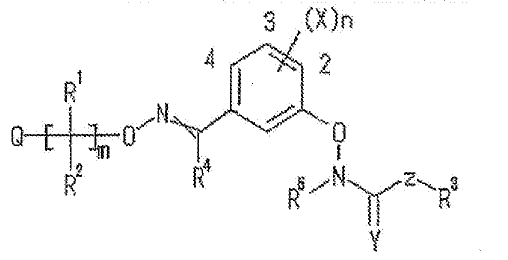 | | | | | | | |

| Compound No. | Q | [C(R¹)(R²)]m | R⁴ | R⁵ | (X)ₙ | Y | Z-R³ |
|---|---|---|---|---|---|---|---|
| 16-1 | 2-MeO-Ph | CH₂ | Me | H | 2-Cl | O | OCH=CH₂ |
| 16-2 | 5-Me-3-MeO-Fh | CH₂ | Me | H | Z-Cl | O | OCH=CH₂ |
| 16-3 | 2-Me-4-MeO-Ph | CH₂ | Me | H | 2-Cl | O | OCH=CH₂ |
| 16-4 | 4-Me-2-MeS-Ph | CH₂ | Me | H | 2-Cl | O | OCH=CH₂ |
| 16-5 | 4-Me-2-MeS(=O)-Ph , | CH₂ | Me | H | 2-Cl | O | OCH=CH₂ |
| 16-6 | 4-Me-2-MeS(=O)₂-Ph | CH₂ | Me | H | 2-Cl | O | OCH=CH₂ |
| 16-7 | 5-Me-3-HeS-Ph | CH₂ | Me | H | 2-Cl | O | OCH=CH₂ |
| 16-8 | 5-Me-3-MeS(=O)-ph | CH₂ | Me | H | 2-Cl | O | OCH=CH₂ |
| 16-9 | 5-Me-3-MeS(=O)-ph | CH₂ | Me | H | 2-Cl | O | OCH=CH₂ |
| 16-10 | 2-N≡C-4-Me-ph | CH₂ | Me | H | 2-Cl | O | OCH=CH₂ |
| 16-11 | 2-N≡C-5-Me-Ph | CH₂ | Me | H | 2-Cl | O | OCH=H₂ |
| 16-12 | 2-NO₂-4-Me-ph | CH₂ | Me | H | 2-Cl | O | OCH=CH₂ |
| 16-13 | 2-NO₂-5-Me-Ph | CH₂ | Me | H | 2-Cl | O | OCH=CH₂ |
| 16-14 | 2-EtOC (=O)-ph | CH₂ | Me | H | 2-Cl | O | OCH=CH₂ |
| 16-15 | 2-EtOC(=O)-5-Me-ph | CH₂ | Me | H | 2-Cl | O | OCH=CH₂ |
| 16-16 | 2,4-(NO₂)₂Ph | CH₂ | Me | H | 2-Cl | O | OCH=CH₂ |
| 16-17 | Ph | CH₂ | Me | H | 2-Cl | O | OCH=CH₂ |
| 16-18 | Np | CH₂ | Me | H | 2-Cl | O | OCH=CH₂ |

[Table 20]

**TABLE 16**

| | | | | | | | |
|---|---|---|---|---|---|---|---|
| 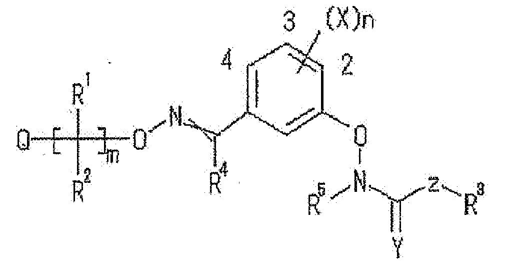 | | | | | | | |

| Compound No. | Q | [C(R¹)(R²)]m | R⁴ | R⁵ | (X)n | Y | Z-R³ |
|---|---|---|---|---|---|---|---|
| 17-1 | 2-Me-Ph | [CH(CH₃)] | Me | H | 2-Cl | O | OMe |
| 17-2 | 3-Me-Ph | [CH(CH₃)] | Me | H | 2-Cl | O | OMe |
| 17-3 | 4-Me-Ph | [CH(CH₃)] | Me | H | 1-Cl | O | OMe |
| 17-4 | 2-Cl-Ph | [CH(CH₃)] | Me | H | 2-Cl | O | OMe |
| 17-5 | 3-Cl-Ph | [CH(CH₃)] | Me | H | 2-Cl | O | OMe |
| 17-6 | 4-Cl-Ph | [CH(CH₃)] | Me | H | 2-Cl | O | OMe |
| 17-7 | 2-MeO-Ph | [CH(CH₃)] | Me | H | 2-Cl | O | OMe |
| 17-8 | 3-MeO-Ph | [CH(CH₃)] | Me | H | 2-Cl | O | OMe |
| 17-9 | 2-MeO-3-Me-Ph | [CH(CH₃)] | Me | H | 2-Cl | O | OMe |
| 17-10 | 2-MeO-4-Me-Ph | [CH(CH₃)] | Me | H | 2-Cl | O | OMe |
| 17-11 | 2-Me-Ph | (CH₂)₄ | Me | H | 2-Cl | O | OMe |
| 17-12 | 3-Me-Ph | (CH₂)₄ | Me | H | 2-Cl | O | OMe |
| 17-13 | 4-Me-Ph | (CH₂)₄ | Me | H | 2-Cl | O | OMe |
| 17-14 | 2-Cl-Ph | (CH₂)₄ | He | H | 2-Cl | O | OMe |
| 17-15 | 3-Cl-Ph | (CH₂)₄ | Me | H | 2-Cl | O | OMe |

[Table 21]

**TABLE 16-(continued)**

| Compound No. | Q | [C(R¹)(R²)]m | R⁴ | R⁵ | (X)n | Y | Z-R³ |
|---|---|---|---|---|---|---|---|
| 17-16 | Ph | 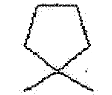 | Me | H | 2-Cl | O | OMe |
| 17-17 | 2,4-Cl₂-Ph | 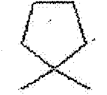 | Me | H | 2-Cl | O | OMe |
| 17-18 | 2-Me-Ph | 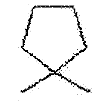 | Me | H | 2-Cl | O | OMe |
| 17-19 | 2-Me-4-MeO-Ph | 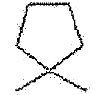 | Me | H | 2-Cl | O | OMe |
| 17-20 | 2-MeO-Ph | 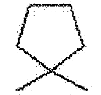 | Me | H | 2-Cl | O | OMe |

[Table 22]

**TABLE 17**

| | | | | | | | |
|---|---|---|---|---|---|---|---|
| 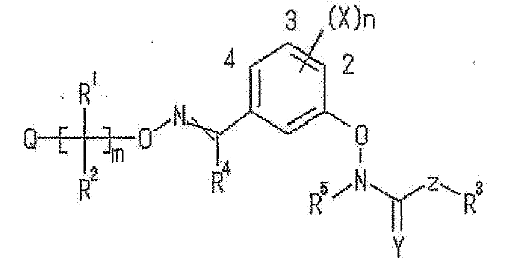 | | | | | | | |

| Compound | Q | [C(R¹)(R²)]m | R⁴ | R⁵ | (X)n | Y | Z-R³ |
|---|---|---|---|---|---|---|---|
| 18-1 | Ph | - | Me | H | 2-Cl | S | OMe |
| 18-2 | Ph | CH₂ | Me | H | 2-Cl | S | OMe |
| 18-3 | 2-Cl-Ph- | CH₂ | Me | H | 2-Cl | S | OMe |
| 18-4 | 3-Cl-Ph | CH₂ | Me | H | 2-Cl | S | OMe |
| 18-5 | 4-Cl-Ph | CH₂ | Me | H | 2-Cl | S | OMe |
| 18-6 | 2-Br-Ph | CH₂ | Me | H | 2-Cl | S | OMe |
| 18-7 | 3-MeS(=O)-Ph | CH₂ | Me | H | 2-Cl | S | OMe |
| 18-8 | 2-MeS(=O)₂-Ph | CH₂ | Me | H | 2-Cl | S | OMe |
| 18-9 | 3-MeS-Ph | CH₂ | Me | H | 2-Cl | S | OMe |
| 18-10 | 3-MeS(=O)-Ph | CH₂ | Me | H | 2-Cl | S | OMe |
| 18-11 | 3-MeS(=O)₂-Ph | CH₂ | Me | H | 2-Cl | S | OMe |
| 18-12 | 2-MeO-Ph | CH₂ | Me | H | 2-Cl | S | OMe |
| 18-13 | 3-MeO-Ph | CH₂ | Me | H | 2-Cl | S | OMe |
| 19-14 | 4-MeO-Ph | CH₂ | Me | H | 2-Cl | S | OMe |
| 18-15 | 2-MeS-Ph | CH₂ | Me | H | 2-Cl | S | OMe |
| 18-16 | 2-MeS(=O)-Ph | CH₂ | Me | H | 2-F | S | OEt |
| 18-17 | 2-MeS(=O)₂₋Ph | CH₂ | Me | H | 2-F | S | OEt |
| 18-18 | 3-MeS-Ph | CHz | Me | H | 2-F | S | OEt |
| 18-19 | 3-MeS(=O)-Ph | CH₂ | Me | H | 2-F | S | OEt |
| 18-20 | 3-MeS(=O)₂-Ph | CH₂ | Me | H | 2-F | S | OEt |

[Table 23]

**TABLE 18**

| | | | | | | | |
|---|---|---|---|---|---|---|---|
| 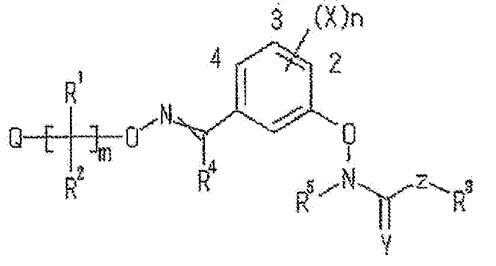 | | | | | | | |

| Compound No. | Q | [C(R¹)(R²) | R⁴ | R⁵ | (X)n | Y | Z-R³ |
|---|---|---|---|---|---|---|---|
| 19-1 | 2-Me-Ph | CH₂ | C≡N | H | 2-Cl | O | OMe |
| 19-2 | 2-MeO-Pb | CH₂ | C≡N | H | 2-Cl | O | OMe |
| 19-3 | 2,4-Me₂-Ph | CH₂ | C≡N | H | 2-Cl | O | OMe |
| 19-4 | 2-MeO-Ph | CH₂ | C≡N | H | 2-Cl | O | OMe |
| 19-5 | 4-Me-2-MeO-ph | CH₂ | C≡N | H | 2-Cl | O | OMe |
| 19-6 | 2-Me-Ph | CH₂ | NH₂ | H | 2-Cl | O | OMe |
| 19-7 | 2-Me-Ph | CH₂ | Me | H | 2-CHF₂ | O | OMe |
| 19-8 | 2-Me-Ph | CH₂ | Me | H | 2-Cl-3-F | O | OMe |
| 19-9 | 3-MeC(=O)-Ph | CH₂ | CHF₂ | H | 2-Cl | S | OMe |
| 19-10 | 3-MeC(=O)-Ph | CH₂ | CF₃ | H | 2-Cl | O | OMe |

[Table 24]

**TABLE 19**

| | | | | | | | |
|---|---|---|---|---|---|---|---|
| 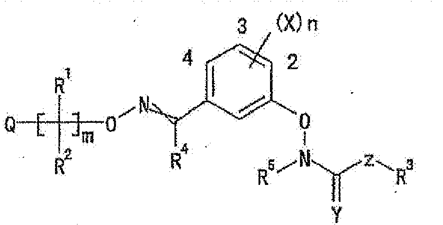 | | | | | | | |

| Compound N° | Q | [C(R¹)(R²)]ₘ | R⁴ | R⁵ | (X)n | Y | Z-R³ |
|---|---|---|---|---|---|---|---|
| 21-1 | Ph | CH₂ | Me | H | 2-Me | O | OMe |
| 21-2 | 3-CF₃-Ph | CH₂ | Me | H | 2-Me | O | OMe |
| 21-3 | 4-CF₃-Ph | CH₂ | Me | H | 2-Me | O | OMe |
| 21-4 | 2-F-Ph | CH₂ | Me | H | 2-Me | O | OMe |
| 21-5 | 3-F-Ph | CH₂ | Me | H | 2-Me | O | OMe |
| 21-6 | 4-F-Ph | CH₂ | Me | H | 2-Me | O | OMe |
| 21-7 | 2-CF₃O-Ph | CH₂ | Me | H | 2-Me | O | OMe |
| 21-8 | 3-CF₃O-Ph | CH₂ | Me | H | 2-Me | O | OMe |
| 21-9 | 2-F-3-CF₃-Ph | CH₂ | Me | H | 2-Me | O | OMe |
| 21-10 | 2-F-4-CF₃-Ph | CH₂ | Me | H | 2-Me | O | OMe |
| 21-11 | 2-F-5-CF₃-Ph | CH₂ | Me | H | 2-Me | O | OMe |
| 21-12 | 2-F-6-CF₃-Ph | CH₂ | Me | H | 2-Me | O | OMe |
| 21-13 | 3-F-2-CF₃-Ph | CH₂ | Me | H | 2-Me | O | OMe |
| 21-14 | 3-F-4-CF₃-Ph | CH₂ | Me | H | 2-Me | O | OMe |
| 21-15 | 3-F-5-CF₃-Ph | CH₂ | Me | H | 2-Me | O | OMe |
| 21-16 | 3-P-6-CF₃-Ph | CH₂ | Me | H | 2-Me | O | OMe |
| 21-17 | 4-F-2-CF₃-Ph | CH₂ | Me | H | 2-Me | O | OMe |
| 21-18 | 4-P-3-CF₃-Ph | CH₂ | Me | H | 2-Me | O | OMe |
| 21-19 | 4-F-5-CF₃-Ph | CH₂ | Me | H | 2-Me | O | OMe |
| 21-20 | 4-F-6-CF₃-Ph | CH₂ | Me | H | 2-Me | O | OMe |
| 21-21 | 2-Et-Ph | CH₂ | Me | H | 2-Me | O | OMe |
| 21-22 | 3-Et-Ph | CH₂ | Me | H | 2-Me | O | OMe |
| 21-23 | 3-Et-2-F-Ph | CH₂ | Me | H | 2-Me | O | OMe |
| 21-24 | 2-CF₃-Ph | CH₂ | Me | H | 2-Me | O | OMe |
| 21-25 | 1-(2,2,2-Trffluoro-ethyl)-piperidin-2-yl | CH₂ | Me | H | 2-Me | O | OMe |
| 21-26 | 1-(2,2,2,-Trifluoro-ethyl)-piperidin-2-yl | CH₂ | Me | AcOCH₂ | 2-Me | O | OMe |
| 21-27 | 3-CF₃-Ph | [CH(C₂H₅)] | Me | H | 2-Me | O | OMe |
| 21-28 | 2-CF₃O-Ph | CH₂ | Me | AcOCH₂ | 2-Me | O | OMe |

[Table 25]

**TABLE 20**

| | | | | | | | |
|---|---|---|---|---|---|---|---|
| 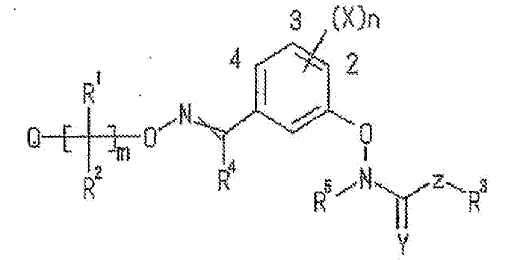 | | | | | | | |

| Compound No. | Q | [C(R¹)(R²)]m | R⁴ | R⁵ | (X)ₙ | Y | Z-R³ |
|---|---|---|---|---|---|---|---|
| 22-1 | 2,3-F₂-Ph | CH₂ | Me | H | 2-Me | O | OMe |
| 22-2 | 2,3,4-F₃-Ph | CH₂ | Me | H | 2-Me | O | OMe |
| 22-3 | 2-FCH₂CH₂-Ph | CH₂ | Me | H | 2-Me | O | OMe |
| 22-4 | 3-FCH₂CH₂-Ph | CH₂ | Me | H | 2-Me | O | OMe |
| 22-5 | 4-FCH₂CH₂-Ph | CH₂ | Me | H | 2-Me | O | OMe |
| 22-6 | 2,4-(CF₃O)₂-Ph | CH₂ | Me | H | 2-Me | O | OMe |
| 22-7 | 2,3-(CF₃O)₂-Ph | CH₂ | Me | H | 2-Me | O | OMe |
| 22-8 | 3-CF₃-Np | CH₂ | Me | H | 2-Me | O | OMe |
| 22-9 | 4-CF₃-Np | CH₂ | Me | H | 2-Me | O | OMe |
| 22-10 | 3,4-CF₃2-Np | CH₂ | Me | H | 2-Me | O | OMe |
| 22-11 | 2-F-1-Np | CH₂ | Me | H | 2-Me | O | OMe |
| 22-12 | 3-F-1-Np | CH₂ | Me | H | 2-Me | O | OMe |
| 22-13 | 2,3-F₃-1-Np | CH₂ | Me | H | 2-Me | O | OMe |
| 22-14 | 2,3,4-F₃-1-Np | CH₃ | Me | H | 2-Me | O | OMe |
| 22-15 | 2-Me-3-CF₃-Ph | CH₂ | Me | H | 2-Me | O | OMe |
| 22-16 | 2-Me-4-CF₃-Ph | CH₂ | Me | H | 2-Me | O | OMe |
| 22-17 | 2-Me-5-CF₃-Ph | CH₂ | Me | H | 2-Me | O | OMe |
| | 22-18 2-Me-6-CF₃-Ph | CH₂ | Me | H | 2-Me | O | OMe |
| 22-19 | 3-Me-2-CF₃-Ph | CH₂ | Me | H | 2-Me | O | OMe |
| 22-20 | 3-Me-4-CF₃-Pn | CH₂ | Me | H | 2-Me | O | OMe |

[Table 26]

**TABLE 21**

| | | | | | | | |
|---|---|---|---|---|---|---|---|
| 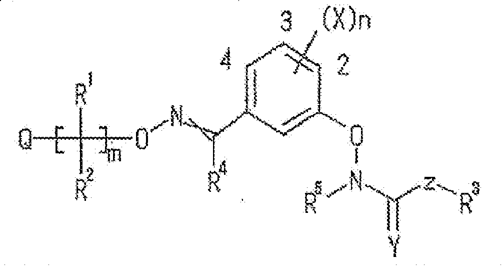 | | | | | | | |

| Compound No. | Q | [C(R¹)(R²)]ₘ | R⁴ | R⁵ | (X)n | Y | Z-R³ |
|---|---|---|---|---|---|---|---|
| 23-1 | 2-CF₃-Ph | CH₂ | Me | CHO | 2-Me | O | OMe |
| 23-2 | 3-CF₃-Ph | CH₂ | Me | CHO | 2-Me | O | OMe |
| 23-3 | 4-CF₃Ph | CH₂ | Me | CHO | 2-Me | O | OMe |
| 23-4 | 2-F-Ph | CH₂ | Me | CHO | 2-Me | O | OMe |
| 23-5 | 3-F-Ph | CH₂ | Me | CHO | 2-Me | O | OMe |
| 23-6 | 4-F-Ph | CH₂ | Me | CHO | 2-Me | O | OMe |
| 23-7 | 2-CF₃O-Ph | CH₂ | Me | CHO | 2-Me | O | OMe |
| 23-8 | 3-CF₃O-Ph | CH₂ | Me | CHO | 2-Me | O | OMe |
| 23-9 | 2-F-3-CF₃-Ph | CH₂ | Me | CHO | 2-Me | O | OMe |
| 23-10 | 2-F-4-CF₃-Ph | CH₂ | Me | CHO | 2-Me | O | OMe |
| 23-11 | 2-F-5-CF₃-Ph | CH₂ | Me | CHO | 2-Me | O | OMe |
| 23-12 | 2-F-6-CF₃-Ph | CH₂ | Me | CHO | 2-Me | O | OMe |
| 23-13 | 3-P-2-CF₃-Ph | CH₂ | Me | CHO | 2-Me | O | OMe |
| 23-14 | 3-F-4-CF₃-Ph | CH₂ | Me | CHO | 2-Me | O | OMe |
| 23-15 | 3-F-5-CF₃-Ph | CH₂ | Me | CHO | 2-Me | O | OMe |
| 23-16 | 3-F-6-CF₃-Ph | CH₂ | Me | CHO | 2-Me | O | OMe |
| 23-17 | 4-F-2-CF₃-Ph | CH₂ | Me | CHO | 2-Me | O | OMe |
| 23-18 | 4-F-3-CF₃-Ph | CH₂ | Me | CHO | 2-Me | O | OMe |
| 23-19 | 4-F-5-CF₃-Ph | CH₂ | Me | CHO | 2-Me | O | OMe |
| 23-20 | 4-F-6-CF₃-Ph | CH₂ | Me | CHO | 2-Me | O | OMe |

[Table 27]

**TABLE 22**

| | | | | | | | |
|---|---|---|---|---|---|---|---|
| 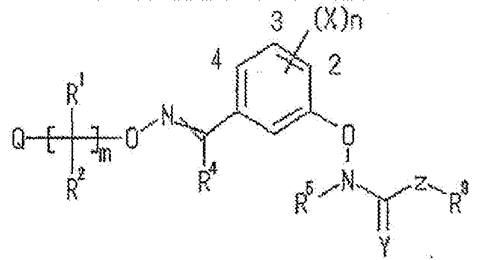 | | | | | | | |

| Compound No. | Q | [C(R¹)(R²)]m | R⁴ | R⁵ | (X)n | Y | Z-R³ |
|---|---|---|---|---|---|---|---|
| 24-1 | 2-CF₃-Ph | CH₂ | Me | H | 2-Et-3-F | O | OMe |
| 24-2 | 3-CF₃-Ph | CH₂ | Me | H | 2-Et-3-F | O | OMe |
| 24-3 | 4-CF₃-Ph | CH₂ | Me | H | 2-Et-3-F | O | OMe |
| 24-4 | 2-F-Ph | CH₂ | Me | H | 2-Et-3-F | O | OMe |
| 24-5 | 3-F-Ph | CH₂ | Me | H | 2-Et-3-F | O | OMe |
| 24-6 | 4-F-Ph | CH₂ | Me | H | 2-Et-3-F | O | OMe |
| 24-7 | 2-CF₃O-Ph | CH₂ | Me | H | 2-Et-3-F | O | OMe |
| 24-8 | 3-CF₃O-Ph | CH₂ | Me | H | 2-Et-3-F | O | OMe |
| 24-9 | 2-F-3-CF₃-Ph | CH₂ | Me | H | 2-Et-3-F | O | OMe |
| 24-10 | 2-F-4-CF₃-Ph | CH₂ | Me | H | 2-Et-3-F | O | OMe |
| 24-11 | 2-F-5-CF₃-Ph | CH₂ | Me | H | 2-Me-3-F | O | OMe |
| 24-12 | 2-F-6-CF₃-Ph | CH₂ | Me | H | 2-Me-3-F | O | OMe |
| 24-13 | 3-F-2-CF₃-Ph | CH₂ | Me | H | 2-Me-3-F | O | OMe |
| 24-14 | 3-F4-CF₃-Ph | CH₂ | Me | H | 2-Me-3-F | O | OMe |
| 24-15 | 3-F-5-CF₃-Ph | CH₂ | Me | H | 2-Me-3-F | O | OMe |
| 24-16 | 3-F-6-CF₃-Ph | CH₂ | Me | H | 2-Me-3-F | O | OMe |
| 24-17 | 4-F-2-CF₃-Ph | CH₂ | Me | H | 2-Me-3-F | O | OMe |
| 24-18 | 4-F-3-CF₃-Ph | CH₂ | Me | H | 2-Me-3-F | O | OMe |
| 24-19 | 4-F-5-CF₃-Ph | CH₂ | Me | H | 2-Me-3-F | O | OMe |
| 24-20 | 4-F-6-CF₃-Ph | CH₂ | Me | H | 2-Me-3-F | O | OMe |

[Table 28]

**TABLE 23**

| | | | | | | | |
|---|---|---|---|---|---|---|---|
| 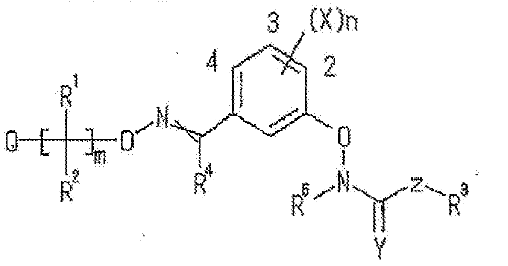 | | | | | | | |

| Compound No. | Q | [C(R¹)(R²)]m | R⁴ | R⁵ | (X)n | Y | Z-R³ |
|---|---|---|---|---|---|---|---|
| 25-1 | 2-CF₃-Ph | (CH₂)₂ | Me | H | 2-Et | O | OMe |
| 25-2 | 3-CF₃-Ph | (CH₂)₂ | Me | H | 2-Et | O | OMe |
| 25-3 | 4-CF₃-Ph | (CH₂)₂ | Me | H | 2-Et | O | OMe |
| 25-4 | 2-F-Ph | (CH₂)₂ | Me | H | 2-Et | O | OMe |
| 25-5 | 3-F-Ph | (CH₂)₂ | Me | H | 2-Et | O | OMe |
| 25-6 | 4-F-Ph | (CH₂)₂ | Me | H | 2-Et | O | OMe |
| 25-7 | 2-CF₃O-Ph | (CH₂)₂ | Me | H | 2-Et | O | OMe |
| 25-8 | 3-CF₃O-Ph | (CH₂)₂ | Me | H | 2-Et | O | OMe |
| 25-9 | 2-F-3-CF₃-Ph | (CH₂)₂ | Me | H | 2-Et | O | OMe |
| 25-10 | 2-F-4-CF₃-Ph | (CH₂)₂ | Me | H | 2-Et | O | OMe |
| 25-11 | 2-F-5-CF₃-Ph | (CH₂)₂ | Me | H | 2-Me | O | OMe |
| 25-12 | 2-F-6-CF₃-Ph | (CH₂)₂ | Me | H | 2-Me | O | OMe |
| 25-13 | 3-F-2-CF₃-Ph | (CH₂)₂ | Me | H | 2-Me | O | OMe |
| 25-14 | 3-F-4-CF₃-Ph | (CH₂)₂ | Me | H | 2-Me | O | OMe |
| 25-15 | 3-F-5-CF₃-Ph | (CH₂)₂ | Me | H | 2-Me | O | OMe |
| 25-16 | 3-F-6-CF₃-Ph | (CH₂)₂ | Me | H | 2-Me | O | OMe |
| 25-17 | 4-F-2-CF₃-Ph | (CH₂)₂ | Me | H | 2-Me | O | OMe |
| 25-18 | 4-F-3-CF₃-Ph | (CH₂)₂ | Me | H | 2-Me | O | OMe |
| 25-19 | 4-F-5-CF₃-Ph | (CH₂)₂ | Me | H | 2-Me | O | OMe |
| 25-20 | 4-F-6-CF₃-Ph | (CH₂)₂ | Me | H | 2-Me | O | OMe |

[Table 29]

**TABLE 24**

| | | | | | | | |
|---|---|---|---|---|---|---|---|
| 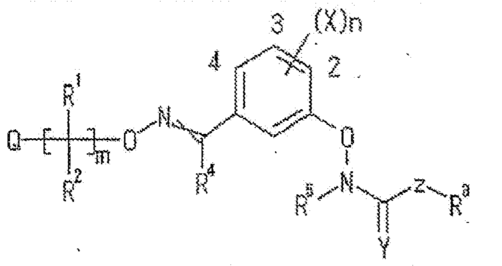 | | | | | | | |

| Compound | Q | [C(R¹)(R²)]m | R⁴ | R⁵ | (X)n | Y | Z-R³ |
|---|---|---|---|---|---|---|---|
| 26-1 | 2-CF₃-Ph | CH₂ | Me | H | 2-Me | O | OCH=CH₂ |
| 26-2 | 3-CF₃-Ph | CH₂ | Me | H | 2-Me | O | OCH=CH₂ |
| 26-3 | 4-CF₃-Ph | CH₂ | Me | H | 2-Me | O | OCH=CH₂ |
| 26-4 | 2-F-Ph | CH₂ | Me | H | 2-Me | O | OCH=CH₂ |
| 26-5 | 3-F-Ph | CH₂ | Me | H | 2-Me | O | OCH=CH₂ |
| 26-6 | 4-F-Ph | CH₂ | Me | H | 2-Me | O | OCH=CH₂ |
| 26-7 | 2-CF₃O-Ph | CH₂ | Me | H | 2-Me | O | OCH=CH₂ |
| 26-8 | 3-CF₃O-Ph | CH₂ | Me | H | 2-Me | O | OCH=CH₂ |
| 26-9 | 2-F-3-CF₃-Ph | CH₂ | Me | H | 2-Me | O | OCH=CH₂ |
| 26-10 | 2-F-4-CF₃Ph | CH₂ | Me | H | 2-Et | O | OCH=CH₂ |
| 26-11 | 2-F-5-CF₃-Ph | CH₂ | Me | H | 2-Et | O | OCH=CH₂ |
| 26-12 | 2-F-6-CF₃-Ph | CH₂ | Me | H | 2-Et | O | OCH=CH₂ |
| 26-13 | 3-F-2-CF₃-Ph | CH₂ | Me | H | 2-Et | O | OCH=CH₂ |
| 26-14 | 3-F4-CF₃-Ph | CH₂ | Me | H | 2-Et | O | OCH=CH₂ |
| 26-15 | 3-F-5-CF₃-Ph | CH₂ | Me | H | 2-Et | O | OCH=CH₂ |
| 26-16 | 3-F-6-CF₃-Ph | CH₂ | Me | H | 2-Bu | O | OCH=CH₂ |
| 26-17 | 4-F-2-CF₃-Ph | CH₂ | Me | H | 2-Bu | O | OCH=CH₂ |
| 26-18 | 4-F-3-CF₃-Ph | CH₂ | Me | H | 2-Bu | O | OCH=CH₂ |
| 26-19 | 4-F-5-CF₃-Ph | CH₂ | Me | H | 2-Bu | O | OCH=CH₂ |
| 26-20 | 4-F-6-CF₃-Ph | CH₂ | Me | H | 2-Bu | O | OCH=CH₂ |

[Table 30]

**TABLE 25**

| | | | | | | | |
|---|---|---|---|---|---|---|---|
| 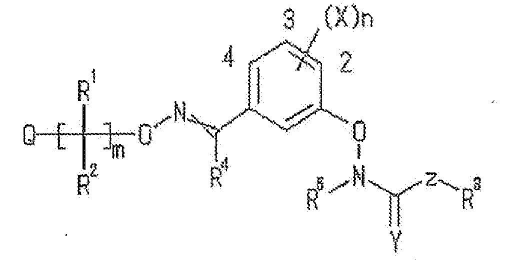 | | | | | | | |

| Compound No. | a | [C(R¹)(R²)]m | R⁴ | R⁵ | (X)n | Y | Z-R³ |
|---|---|---|---|---|---|---|---|
| 274 | 2-CF₃-Ph | [CH(CH₃)] | Me | H | 2-Me | O | OMe |
| 27-2 | 3-CF₃-Ph | [CH(CM₃)] | Me | H | 2-Me | O | OMe |
| 27-3 | 4-CF₃-Ph | [CH(CH₃)] | Me | H | 2-Me | O | OMe |
| 27-4 | 2-F-Ph | [CH(CH₃)] | Me | H | 2-Me | O | OMe |
| 27-5 | 3-F-Ph | [CB(CH₃)] | Me | H | 2-Me | O | OMe |
| 27-6 | 4-F-Ph | [CH(CH₃)] | Me | H | 2-Me | O | OMe |
| 27-7 | 2-CF₃O-Ph | [CH(CH₃)] | Me | H | 2-Me | O | OMe |
| 27-8 | 3-CF₃O-Ph | [CH(CH₃)] | Me | H | 2-Me | O | OMe |
| 27-9 | 2-F-3-CF₃-Ph | [CH(CH₃)] | Me | H | 2-Me | O | OMe |
| 27-10 | 2-F-4-CF₃-Ph | [CH(CH₃)] | Me | H | 2-Me | O | OMe |

[Table 31]

**TABLE 25-(continued)**

| Compound No. | Q | [C(R¹)(R²)]m | R⁴ | R⁵ | (X)n | Y | Z-R³ |
|---|---|---|---|---|---|---|---|
| 27-16 | 2-CF₃-Ph | 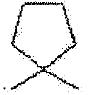 | Me | H | 2-Me | O | OMe |
| 27-17 | 2-F-3-CF₃O-Ph | 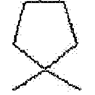 | Me | H | 2-Me | O | OMe |
| 27-18 | 2-CF₃-Ph | 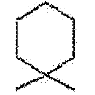 | Me | H | 2-Me | O | OMe |
| 27-19 | 2-F-3-CF₃O-Ph | 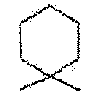 | Me | H | 2-Me | O | OMe |
| 27-20 | 3-CF₃O-Ph | 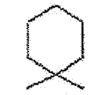 | Me | H | 2-Me | O | OMe |

[Table 32]

**TABLE 26**

| | | | | | | | |
|---|---|---|---|---|---|---|---|
| 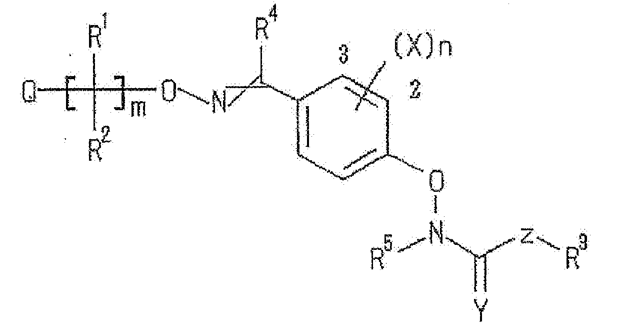 | | | | | | | |

| Compound No. | Q | [C(R¹)(R²)]m | R⁴ | R⁵ | (X)n | Y | Z-R³ |
|---|---|---|---|---|---|---|---|
| 27-11 | 2-CF₃-Ph | (CH₂)₄ | Me | H | 2-Me | O | OMe |
| 27-12 | 3-CF₃-Ph | (CH₂)₄ | Me | H | 2-Me | O | OMe |
| 27-13 | 4-CF₃-Ph | (CH₂)₄ | Me | H | 2-Me | O | OMe |
| 27-14 | 2-F-Ph | (CH₂)₄ | Me | H | 2-Me | O | OMe |
| 27-15 | 3-F-Ph | (CH₂)₄ | Me | H | 2-Me | O | OMe |

[Table 33]

**TABLE 27**

| | | | | | | | |
|---|---|---|---|---|---|---|---|
| 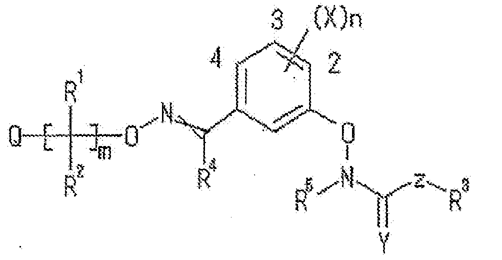 | | | | | | | |

| Compound No. | Q | [C(R¹)(R²)]m | R⁴ | R⁵ | (X)n | Y | Z-R³ |
|---|---|---|---|---|---|---|---|
| 28-1 | 2-CF₃-Ph | CH₂ | Me | H | 2-Me | S | OMe |
| 28-2 | 3-CF₃-Ph | CH₂ | Me | H | 2-Me | S | OMe |
| 28-3 | 4-CF₃-Ph | CH₂ | Me | H | 2-Me | S | OMe |
| 28-4 | 2-F-Ph | CH₂ | Me | H | 2-Me | S | OMe |
| 28-5 | 3-F-Ph | CH₂ | Me | H | 2-Me | S | OMe |
| 28-6 | 4-F-Ph | CH₂ | Me | H | 2-Me | S | OMe |
| 29-7 | 2-CF₃O-Ph | CH₂ | Me | H | 2-Me | S | OMe |
| 28-8 | 3-CF₃O-Ph | CH₂ | Me | H | 2-Me | S | OMe |
| 28-16 | 3-CF₃-Ph | CH₂ | Me | H | 2-Me-4-F | O | OEt |
| 28-17 | 4-CF₃-Ph | CH₂ | Me | H | 2-Me-4-F | O | OEt |
| 28-18 | 2-F-Ph | CH₂ | Me | H | 2-Me-4-F | O | OEt |
| 28-19 | 3-F-Ph | CH₂ | Me | H | 2-Me-4-F | O | OEt |
| 28-20 | 3-CF₃O-Ph | CH₂ | Me | H | 2-Me-4-F | O | OEt |

[Table 34]

**TABLE 28**

| | | | | | | | |
|---|---|---|---|---|---|---|---|
| 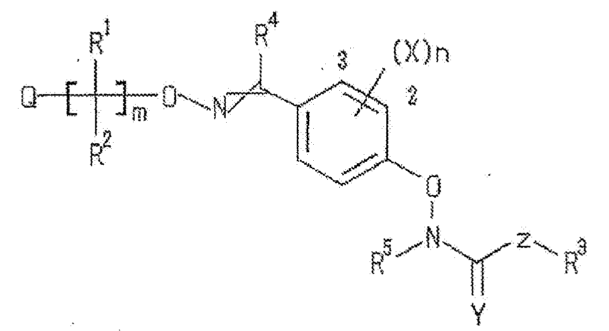 | | | | | | | |

| Compound No. | Q | [C(R¹)(R²)]m | R⁴ | R⁵ | (X)n | Y | Z-R³ |
|---|---|---|---|---|---|---|---|
| 28-9 | 2-CF₃C(=O)-Ph | CH₂ | Me | H | 2-Me | S | OMe |
| 28.10 | 3-C-F₃C(=O)-Ph | CH₂ | Me | H | 2-Me | O | OMe |
| 28-11 | 4-CF₃C(=O)-Ph | CH₂ | Me | H | 2-Me | O | OMe |
| 28-12 | 3-FCH₂CH₂O-Ph | CH₂ | Me | H | 2-Me | O | OMe |
| 28-13 | 4-FCH₂CH₂O-Ph | CH₂ | Me | H | 2-Me | O | OMe |
| 28-14 | 3,4-(CF₃O)₂-Ph | CH₂ | Me | H | 2-Me | O | OMe |
| 28-15 | 2,4-(CF₃O)₂-Ph | CH₂ | Me | H | 2-Me | O | OMe |

### 2) Production method

For example, the compound of the present invention may be prepared in the following manner.

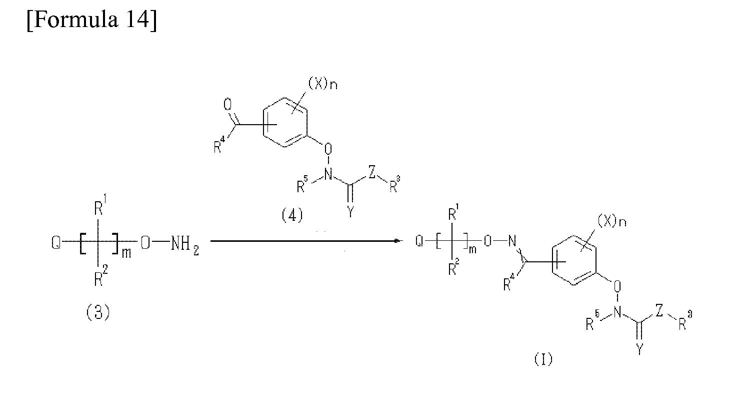

### (wherein R¹ to R⁵, X, Z, Q, Y, m and n are defined as above)

That is, a compound represented by Formula (I) can be obtained by reacting an oxyamine compound represented by Formula (3) with a ketone compound represented by Formula (4) (hereinafter, also referred to as "compound (4)").

The amount of compound (4) used is generally 0.5 to 2 times moles, preferably 0.7 to 1.5 times moles with respect to the oxyamine compound represented by Formula (3).

This reaction may be carried out in the absence of a catalyst, but is preferably carried out in the presence of an acid or base catalyst, more preferably, in the presence of an acid catalyst.

Examples of useful acid catalysts include trifluoroacetic acid, benzenesulfonic acid, p-toluenesulfonic acid, p-toluenesulfonate monohydrate, methanesulfonic acid, pyridinium p-toluenesulfonate, hydrochloric acid, and sulfuric acid. Examples of the base catalyst include pyridine, triethylamine and potassium hydroxide.
The amount of catalyst used is generally 0.0001 to 1times moles with respect to the oxyamine compound represented by Formula (3).

In addition, a dehydrating agent such as anhydrous sodium sulfate, and Molecular sieves may be added in a reaction system.

This reaction may be carried out in a suitable solvent. Any solvent may be used without particular limitation so long as it is inert to the reaction. Examples of the solvent include ether solvents such as dioxane, 1,2-dimethoxyethane, and tetrahydrofuran; aromatic hydrocarbon solvents such as toluene, benzene and xylene; aliphatic hydrocarbon solvents such as n-pentane, n-hexane and n-heptane; halogen-substituted hydrocarbon solvents such as dichloromethane, chloroform, carbon tetrachloride and 1,2-dichloroethane; amide solvents such as N,N-dimethylformamide, N,N-dimethylacetamide and N-methylpyrrolidone; nitrile solvents such as acetonitrile and benzonitrile; alcohol solvents such as methanol, ethanol and n-propanol; and solvent mixtures of two or more thereof.
The amount of solvent used is not particularly limited, and is generally 1 to 100 ml with respect to 1g of oxyamine compound represented by Formula (3).

The reaction temperature ranges from ambient temperature to the boiling point of the solvent used. The reaction period generally ranges from several minutes to several tens of hours.

The oxyamine compound represented by Formula (3) may be prepared by a known method for preparing the oxyamine compound. For example, as depicted below, a compound represented by Formula (I) reacts with carbon tetrabromide and triphenylphosphine to obtain a compound represented by Formula (2), the compound (2) reacts with N-t-butoxycarbonyl hydroxyl amine in the presence of a base to obtain a oxyamine compound represented by Formula (9), and the oxyamine compound (9) reacts with an acid to obtain an oxyamine compound represented by Formula (3).

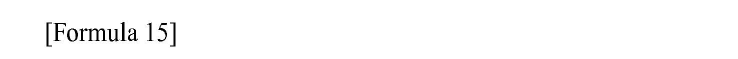

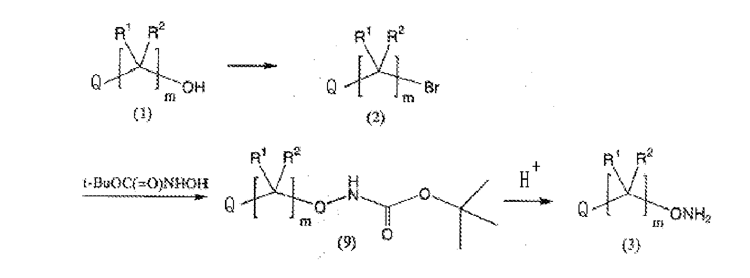

(wherein R¹, R², Q and m are defined as above)
In addition, in the case where the oxyamine compound represented by Formula (3) reacts with the compound (4), the reaction may be carried out by mixing the compound (4) with the oxyamine compound represented by Formula (9), adding an acid such as trifluoroacetic acid thereto and producing the compound represented by Formula (3) in a reaction system.
For example, the compound (4) may be prepared in the following manner.

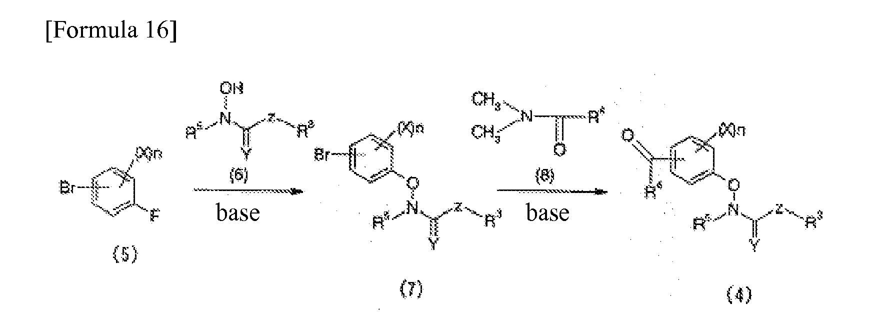

(wherein X, n, R³ to R⁵, Y and Z are defined as above) That is, first, a fluoride compound represented by Formula (5) (hereinafter, also referred to as "compound (5)") reacts with a compound represented by Formula (6) (hereinafter, also referred to as "compound (6)") in the presence of a base to obtain a hydroxycarbamic acid compound represented by Formula (7) (hereinafter, also referred to as "compound (7)"). Then, the compound (7) reacts with an amide compound represented by Formula (8) (hereinafter, also referred to as "compound (8)") in the presence of a base to obtain the desired compound (4).

In the reaction for obtaining the compound (7), the amount of compound (6) used is generally 0.8 to 5 times moles, preferably 1 to 3 times moles, with respect to one mole of the compound (5).

Examples of the base used for reaction for obtaining the compound (7) include metal hydroxides such as lithium hydroxide, sodium hydroxide, potassium hydroxide, magnesium hydroxide, calcium hydroxide; metal alkoxides such as sodium methoxide, sodium ethoxide, potassium methoxide, potassium ethoxide and potassium t-butoxide; metal hydrides such as sodium hydride, potassium hydride and calcium hydride; and organic bases such as triethylamine, diisopropylethylamine, pyridine, 1,8-diazabicyclo[5.4.0]undece-7-en (DBU), and 1,4-diazabicyclo[2.2.2]octane.
The amount of base used is generally 1 to 20 times moles with respect to one mole of the compound (5).

This reaction may be carried out in a suitable organic solvent. Any organic solvent may be used without particular limitation so long as it is inert to the reaction. Examples of the organic solvent include sulfur-containing solvents such as dimethylsulfoxide and diethylsulfoxide; ether solvents such as dioxane, 1,2-dimethoxyethane and tetrahydrofuran; aromatic hydrocarbon solvents such as toluene, benzene and xylene; aliphatic hydrocarbon solvents such as n-pentane, n-hexane, and n-heptane; amide solvents such as N,N-dimethylformamide, N,N-dimethylacetamide and N-methylpyrrolidone; nitrile solvents such as acetonitrile, benzonitrile; and solvent mixtures of two or more thereof.
The amount of organic solvent used is not particularly limited, but is generally 1 to 100 ml with respect to 1 g of the compound (5).
The reaction for obtaining the compound (7) is smoothly carried out in the temperature range from 0°C to the boiling point of the solvent used.

The specific examples of compound (8) used for the reaction for obtaining the compound (4) include N,N-dimethylacetamide and N,N-dimethyl propionamide.
The amount of compound (8) used is generally 0.8 to 5 times moles, preferably 1 to 3 times moles, with respect to one mole of the compound (7).

Examples of the base used for reaction for obtaining the compound (4) include organic lithium compounds such as n-butyl lithium, sec-butyl lithium, t-butyl lithium and lithium diisopropylamide; alkali metals such as sodium metal, and potassium metal; and metal hydrides such as sodium hydride, potassium hydride and calcium hydride.
The amount of base used is generally 1 to 20 times moles with respect to one mole of the compound (7).

This reaction may be carried out in a suitable organic solvent. Any organic solvent may be used without particular limitation so long as it is inert to the reaction. Examples of the organic solvent include ether solvents such as dioxane, 1,2-dimethoxyethane and tetrahydrofuran; aromatic hydrocarbon solvents such as toluene, benzene and xylene; and aliphatic hydrocarbon solvents such as n-pentane, n-hexane and n-heptane.
The reaction for obtaining the compound (4) is smoothly carried out in the temperature range from -100°C to the boiling point of the solvent used.

Among the compound (4), the compound (4-a) wherein R³ represents a t-butyl group, R⁵ represents a hydrogen atom, and Y and Z represent an oxygen atom may be induced into a compound (4-c) wherein the t-butyl group is changed by another substituent group R^{3'} (R^{3'} has the same definition as R³, except for a t-butyl group) by the following method known in the art.

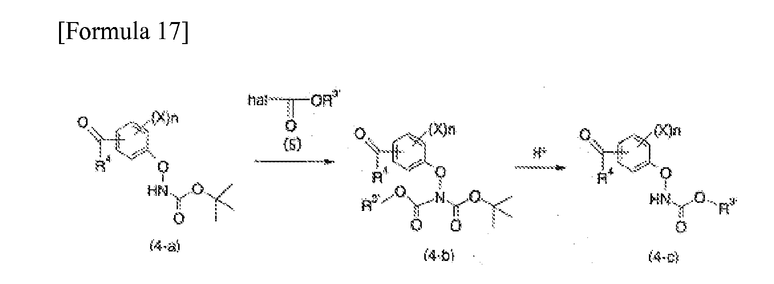

(wherein X, n and R⁴ are defined as above, hal represents a halogen atom such as a chlorine atom and a bromine atom, and R^{3'} has the same definition as R³, except for a t-butyl group)

That is, first, a carbamic acid t-butyl ester compound represented by Formula (4-a) reacts with a halogenoformic acid ester compound represented by Formula (5) in the presence of a base to obtain a compound represented by Formula (4-b). Then, the compound (4-b) reacts with an acid to obtain a compound represented by Formula (4-c).

Examples of the base used for reaction of the halogenoformic acid ester compound represented by Formula (5) include metal hydroxides such as lithium hydroxide, sodium hydroxide, potassium hydroxide, magnesium hydroxide, and calcium hydroxide; metal alkoxides such as sodium methoxide, sodium ethoxide, potassium methoxide, potassium ethoxide and potassium t-butoxide; metal hydrides such as sodium hydride, potassium hydride and calcium hydride; and organic bases such as triethylamine, diisopropylethylamine, pyridine, 1,8-diazabicyclo[5.4.0]undece-7-en (DBU) and 1,4-diazabicyclo[2.2.2]octane.
The amount of base used is generally 1 to 20 times moles with respect to one mole of the compound (4-a).

The reaction of the halogenoformic acid ester compound represented by Formula (5) may be carried out in a suitable organic solvent. Any organic solvent may be used without particular limitation so long as it is inert to the reaction. Examples of the organic solvent include halogen-substituted hydrocarbon solvents such as methylene chloride, chloroform, carbon tetrachloride and 1,2-dichloroethane; sulfur-containing solvents such as dimethylsulfoxide and diethylsulfoxide; ether solvents such as dioxane, 1,2-dimethoxyethane and tetrahydrofuran; aromatic hydrocarbon solvents such as toluene, benzene and xylene; aliphatic hydrocarbon solvents such as n-pentane, n-hexane and n-heptane; amide solvents such as N,N-dimethylformamide, N,N-dimethylacetamide and N-methylpyrrolidone; nitrile solvents such as acetonitrile and benzonitrile; and solvent mixtures of two or more thereof.
The amount of organic solvent used is not particularly limited, but is generally 1 to 100 ml with respect to 1 g of the compound (4-a).

The reaction of the halogenoformic acid ester compound represented by Formula (5) is smoothly carried out in the temperature range from -20°C to the boiling point of the solvent used.
The reaction period depends on the reaction scale and generally ranges from several minutes to several tens of hours.

Examples of acid used for the reaction for obtaining the compound represented by Formula (4-c) from the compound represented by Formula (4-b) include inorganic acids such as hydrochloric acid, sulfuric acid and nitric acid; acetic acid, trifluoroacetic acid, methanesulfonic acid and p-toluenesulfonic acid.
The amount of acid used is generally 1 to 20 times moles with respect to one mole of the compound (4-b).

The reaction for obtaining the compound represented by Formula (4-c) from the compound represented by Formula (4-b) is carried out in a suitable organic solvent. Any organic solvent may be used without particular limitation so long as it is inert to the reaction and examples thereof are the same as examples of solvent used for the reaction of the halogenoformic acid ester compound represented by Formula (5).

The reaction for obtaining the compound represented by Formula (4-c) from the compound represented by Formula (4-b) is smoothly carried out in the temperature range from -20°C to the boiling point of the solvent used.
The reaction period depends on the reaction scale and generally ranges from several minutes to several tens of hours.

In addition, among the compound (4), compound (4-d) wherein Y and Z represent an oxygen atom may be induced into a compound (4-e) wherein the substituent group R³ is changed by another substituent group R^{3''} by an ester exchange method known in the art (depicted by reaction scheme below).

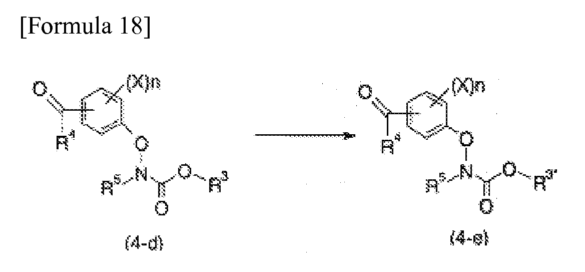

(wherein X, n and R³ to R⁵ are defined as above and R^{3''} has the same definition as R³. R³ is not same as R^{3''})

Any salt of the compound of the present invention may be used without particular limitation so long as it is an agriculturally acceptable salt. Examples of the salt include salts of inorganic acids such as hydrochloric acid and sulfuric acid; and salts of organic acids such as acetic acid and lactic acid.
These salts may be prepared using the oxime ether derivative represented by Formula (I) and corresponding acids by a conventional method known in the art.

In all the reactions, after completion of the reaction, the target product can be separated by a common post-treatment operation and a known conventional purification method such as distillation, recrystallization and column chromatography, if desired.
The structure of the target product can be identified and confirmed by a known conventional analysis method such as IR spectrum, NMR spectrum, mass spectrum and elemental assay.

### 3) Fungicide for agriculture and horticulture

The second aspect of the present invention is a fungicide for agriculture and horticulture containing at least one of the oxime ether derivatives represented by Formula (I) or a salt thereof of the present invention, as an active ingredient (hereinafter, also referred to as "the fungicide of the present invention ").

The fungicide of the present invention contains the compound of the present invention as an active ingredient and exhibits superior sterilizing power in a wide range species of filamentous fungi such as oomycetes, ascomycetes, deuteromycetes and basidiomycetes.

The fungicide of the present invention may be used via seed treatment, foliage application, soil application or water surface application to prevent a variety of disease damage occurring during cultivation of agricultural and horticultural products including flowers, grass and pasture.

For example, the fungicide of the present invention may be used for prevention of the following diseases:

| | |
|---|---|
| Sugar beets | Cercospora beticola |
| | Aphanomyces cochlloides |
| Peanuts | Mycosphaerella arachidis |
| | Mycosphaerella berkeleyi |
| Cucumbers | Sphaerotheca fuliginea |
| | Mycosphaerella melonis |
| | Sclerotinia sclerotiorum |
| | Botrytis cinerea |
| | Cladosporium cucumerinum |
| | Corynespora cassicola |
| | Pythium debaryanam, Rhizoctonia solani Kuhn |
| | Pseudomonas syringae pv. Lecrymans |
| Tomatoes | Botrytis cinerea |
| | Cladosporium fulvum |
| Eggplants | Botrytis cinerea |
| | Corynespora melongenae |
| | Erysiphe cichoracearum |
| | Mycovellosiella nattrassii |
| Strawberries | Botrytis cinerea |
| | Sphaerotheca humuli |
| | Colletotrichum acutatum, Colletotrichum fragariae |
| Onions | Botrytis allii |
| | Botrytis cinerea |
| | Botrytis squamosa |
| Cabbage | Plasmodiophora brassicae |
| | Erwinia carotovora |
| Kidney beans | Sclerotinia sclerotiorum |
| | Botrytis cinerea |
| Apples | Podosphaera leucotricha |
| | Venturia inaequalis |
| | Monilinia mali |
| | Valsa mali |
| | Alternaria mali |
| | Gymnosporangium yamadae |
| | Botryosphaeria berengeriana |
| | Colletotrichum gloeosporioides |
| | Diplocarpon mali |
| Persimmons | Phyllactinia kakicola |
| | Gloeosporium kaki |
| | Cercospora kaki |

Peaches and cherries Monilinia fructicola

| | |
|---|---|
| Grapes | Botrytis cinerea |
| | Uncinula necator |
| | Glomerella cingulata |
| Pears | Venturia nashicola |
| | Gymnosporangium asiaticum |
| | Alternaria kikuchiana |
| Tea | Pestalotia theae |
| | Colletotrichum theae-sinensis |
| Citrus | Elsinoe fawcetti |
| | Penicillium italicum |
| | Penicillium digitatum |
| | Botrytis cinerea |
| | Diaporthe citri |
| | Xanthomonas campestris pv. Citri |
| Wheat | Erysiphe graminis f. sp. tritici |
| | Gibberella zeae |
| | Puccinia recondita |
| | Pythium iwayamai |
| | Monographella nivalis |
| | Pseudocercosporella herpotrichoides |
| | Septoria tritici |
| | Leptosphaeria nodorum |
| | Typhula incamata |
| | Myriosclerotinia borealis |
| | Gaeumanomyces graminis |

| | |
|---|---|
| Barley | Pyrenophora graminea |
| | Rhynchosporium secalis |
| | Ustilago tritici, U. nuda |
| Rice | Pyricularia oryzae |
| | Rhizoctonia solani |
| | Gibberella fujikuroi |
| | Cochliobolus niyabeanus |
| | Pythium graminicolum |
| Tobacco | Sclerotinia sclerotiorum |
| | Erysiphe cichoracearum |
| Tulip | Botrytis cinerea |
| Bent grass | Sclerotinia borealis |
| | Pythium aphanidermatum |
| Orchard Grass | Erysiphe graminis |
| Soybeans | Cercospora kikuchii |
| | Peronospora Manshurica |
| | Phytophthora sojae |
| Sweet potato ·Tomato | Phytophthora infestans |
| Cucumber | Pseudoperonospora cubensis |
| Grape | Plasmopara viticola |

In addition, recently, various pathogenic fungi have exhibited increasing resistance to benzimidazole fungicides or dicarboxyimide fungicides, thus causing these chemicals to have insufficient effects and resulting in a need for chemicals capable of exerting effects on drug-resistant fungi. The fungicide of the present invention exhibits superior fungic killing effects on drug-resistant fungi as well as sensitive pathogenic fungi to the chemicals.

For example, similar to the sensitive fungi, the fungicide of the present invention exerts effects on Botrytis cinerea, Cercospora beticola, Venturia inaequalis and Venturia nashicola, which are resistant to benzimidazole fungicides such as thiophanate methyl, benomyl and carbendazim.

In addition, similar to the sensitive fungi, the fungicide of the present invention exerts effects on Botrytis cinerea resistant to dicarboxyimide fungicides (such as vinclozolin, procymidone and iprodione).

Examples of diseases, to which the present invention applies, more preferably include: Cercospora beticola which damages sugar beets, Erysiphe graminis f sp tritici which damages wheat, Pyricularia oryzae which damages rice, Venturia inaequalis which damages apples, Botrytis cinerea which damages cucumbers, and Mycosphaerella arachidis which damages peanuts.

In addition, the fungicide of the present invention is a chemical which exhibits low phytotoxicity, low toxicity to fish or warm-blooded animals and high safety.
When the fungicide of the present invention is practically applied, the compound of the present invention may be used in a pure form without adding other ingredients, or provided in a general form for agricultural pesticides, that is, a formulation for agricultural pesticides such as wettable powders, granules, dispersible powders, emulsions, agueous solutions, suspensions, water dispersible granules or the like.

Examples of additives and carriers which may be added to the agricultural pesticides include, for solid formulations, vegetable powders such as soybean powder and wheat powder, mineral fine powders such as diatomite, apatite, plaster, talc, bentonite, pyrophyllite, clay, and organic and inorganic compounds such as soda benzoate, urea and salt cake.

In addition, for liquid formulations, a solvent such as kerosene-, xylene- and petroleum-series aromatic hydrocarbon, cyclohexane, cyclohexanone, dimethylformamide, dimethylsulfoxide, alcohol, acetone, trichloroethylene, methylisobutylketone, mineral oils, vegetable oils and water may be used.

In addition, in order to make these formulations homogeneous and stable, a surfactant may be optionally added thereto.
Any surfactant may be added without particular limitation and examples thereof include nonionic surfactants such as polyoxyethylene-added alkylphenyl ethers, poly oxyethylene-added alkyl ethers, poly oxyethylene-added higher fatty acid esters, poly oxyethylene-added sorbitan higher fatty acid esters, and poly oxyethylene-added tristyrylphenyl ethers, sulfuric acid ester salts of polyoxyethylene-added alkylphenyl ethers, alkylbenzenesulfonate, sulfuric acid ester salts of higher alcohols, alkylnaphthalene sulfonates, polycarboxylates, lignin sulfonate, formaldehyde condensates of alkylnaphthalene sulfonates, and isobutylene-anhydrous maleic acid copolymers.

The wettable powder, emusifiable concentrate, flowable formulation, water-soluble formulation, or water dispersible granule thus obtained is diluted with water to a predetermined concentration to provide a solution, suspension or emulsion, and a dispersible powder and granule is sprayed onto plants without any treatment.

The amount of the active ingredient in the fungicide of the present invention is generally preferably 0.01 to 90% by mass, more preferably 0.05 to 85% by mass with respect to the total mass of the composition (formulation).

An amount of the fungicide of the present invention applied may depend on meteorological conditions, formulation type, applied season, applied methods, applied places, disease damage to be presented and target crops, and is generally 1 to 1000 g, preferably 10 to 100 g with respect to the amount of active ingredient compound per hectare.

In the case where the wettable powder, emulsifiable concentrate, suspension, water-soluble formulation and the water dispersible granule is diluted with water, the concentration applied is 1 to 1000 ppm, preferably 10 to 250 ppm.

The fungicide of the present invention may contain, in addition to the compound of the present invention, one or more of a variety of fungicides, insecticides, miticides and synergists.

Representative examples of fungicide, insecticide, miticide and plant growth regulator which can be used as mixture with the compound of the present invention will be described below:

### Fungicides:

captan, folpet, thiuram, ziram, zineb, maneb, mancozeb, propineb, polycarbamate, chlorothalonil, quintozene, captafol, iprodione, procymidone, vinclozolin, fluoroimide, cymoxanil, mepronil, flutolanil, phencyclone, oxycarboxin, fosetyl aluminum, propamocarb, triadimefon, triadimenol, propiconazole, dichlorotriazole, bitertanol, hexaconazole, microbutanyl, flusilazole, metconazole, econazole, fluotrimazole, cyproconazole, epoxiconazole, flutriafen, penconazole, dimiconazole, cyproconazole, fenarimol, triflumizole, prochloraz, imazalil, pefurazoate, tridemorph, fenpropimorph, trifolin, buthiobate, pyrifenox, anilazine, polyoxin, metalaxyl, oxadixyl, furalaxyl, isoprothiolane, probenazole, pyrrolnitrin, blasticidin S, kasugamycin, validamycin, dihydrostreptomycin sulfate, benomyl, carbendazim, thiophanate-methyl, hymexazole, basic copper chloride, basic copper sulfate, pentin acetate, triphenyl tin acetate, triphenyl tin hydroxide, diethofencarb, methasulfocarb, quinomethionate, binapacryl, lecithin, sodium carbonate, dithianon, dinocap, fenaminosulf, dichlomezine, guazatine, dodine, IBP, edifenphos, mepanipyrim, ferimzone, trichlamide, methasulfocarb, fluazinam, etokinorakku, dimethomorph, pyroquilon, tecloftalam, fthalide, phenazineoxide, thiabendazole, tricyclazole, vinclozolin, cymoxanil, myclobutanil, guazatine, propamocarb hydrochloride, oxolinic acid, hydroxyisoxazole, iminoctadine acetate, etc.

### Insecticides·miticides:

### Organophosphorus and carbamate insecticides:

fenthion, fenitrothion, diazinon, chlorpyriphos, ESP, vamidothion, fenthoate, dimethoate, formothion, malathion, trichlorfon, thiometon, phosmet, dichlorvos, acephate, EPBP, methyl parathion, oxydemeton methyl, ethion, salithion, cyanophos, isoxathion, pyridafenthion, phosalone, methidathion, sulprofos, chlorfevinphos, tetrachlorvinphos, dimethylvinphos, propaphos, isofenphos, ethylthiometon, profenophos, pyraclofos, monocrotophos, azinphos methyl, aldicarb, methomyl, thiodicarb, carbofuran, carbosulfan, benfuracarb, furathiocarb, propoxur, BPMC, MTMC, MIPC, carbaryl, pirimicarb, ethiofencarb, fenoxycarb, EDDP, etc.

### Pyrethroid-based insecticides:

permethrin, cypermethrin, deltamethrin, fenvalerate, fenpropathrin, pyrethrin, allethrin, tetramethrin, resmethrin, dimethrin, propathrin, phenothrin, prothrin, fluvalinate, cyfluthrin, cyhalothrin, flucythrinate, ethofenprox, cycloprothrin, tralomethrin, silafluofen, brofenprox, acrinathrin, etc.

### Benzoylphenylureas and other insecticides:

diflubenzuron, chlorfluazuron, hexaflumuron, triflumuron, teflubenzuron, flufenoxuron, flucycloxuron, buprofezin, pyriproxyfen, methoprene, benzoepin, diafenthiuron, acetamiprid, imidacloprid, nitenpyram, fipronil, caltop, thiocyclam, nicotine sulfate, rotenone, metaldehyde, machine oil, bacillus thuringiensis (BT), microbial pesticides such as entomopathogenic viruses, etc.

nematicides:
fenamiphos, fosthiazate, etc.
miticides:
   chlorobenzilate, phenisobromolate, dicofol, amitraz, BPPS, benzomate, hexythiazox, fenbutatin oxide, polynactin, kinomethionate, CPCBS, tetradifon, avermectin, milbemectin, clofentezine, cyhexatin, pyridaben, fenpyroximate, tebufenpyrad, pyrimidifen, phenothiocarb, dienochlor, etc.
   plant growth regulators:
Gibberellins (such as gibberellin A3, gibberellin A4 and gibberellin A7), IAA, NAA, etc.

### [Examples]

Next, the present invention will be described with reference to examples in more detail, but is not limited thereto.

### (Example 1)

Preparation of N-(2-chloro-5- {1-[3-fluoro-2-(trifluoromethyl)benzyloxyimino]ethyl}phenoxy)methyl carbamate (compound 1-13)

### (Process 1)

### N-[3-fluoro-2-(trifluoromethyl)benzyloxy]t-butyl carbamate

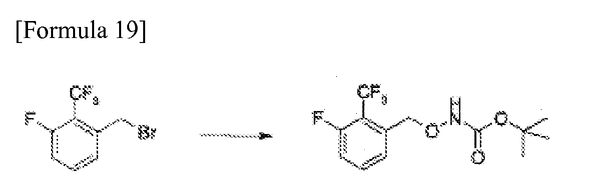

20 ml of acetonitrile, 0.63 g ofN-Boc-hydroxylamine and 0.72 g of 1,8-diazabicyclo[5.4.0]-7-undecene (DBU) were added to 1.00 g of 3-fluoro-2-(trifluoromethyl)benzyl bromide, followed by stirring at ambient temperature for 3 hours. After the completion of reaction, ethyl acetate was added to the reaction mixture and washed with 7% hydrochloric acid and 10% aqueous sodium hydroxide solution in this order. The organic layer thus obtained was dried over magnesium sulfate and filtered and the solvent was distilled under reduced pressure to obtain 1.20 g of the desired crude N-[3-fluoro-2-(trifluoromethyl)benzyloxy]t-butyl carbamate.

### (Process 2)

### Preparation of N-(2-chloro-5- {1-[3-fluoro-2-(trifluoromethyl)benzyloxyimino]ethyl}phenoxy)methyl carbamate

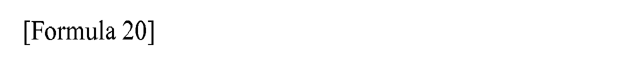

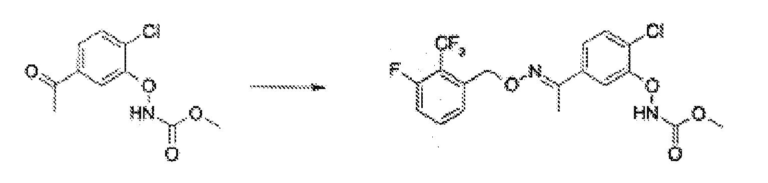

A mixture of 0.30 g ofN-(5-acetyl-2-chlorophenoxy)methyl carbamate and 0.49 g of the crude N-[3-fluoro-2-(trifluoromethyl)benzyloxy]t-butyl carbamate thus obtained was dissolved in 15 ml of dichloroethane, and 1 ml of trifluoroacetic acid was added thereto, followed by stirring at 50°C for one hour. After completion of the reaction, ethyl acetate was added to the reaction mixture, followed by washing with a saturated aqueous sodium bicarbonate solution. An organic layer was separated, dried over magnesium sulfate, filtered, and the solvent was then distilled under reduced pressure. The resulting residue was purified by silica gel column chromatography (eluent; hexane:ethyl acetate=3:1) to obtain 0.43 g of the desired N-(2-chloro-5- {1-[3-fluoro-2-(trifluoromethyl)benzyloxyimino]ethyl}phenoxy)methyl carbamate (yield 80%, melting point: 104 to 105°C, compound 1-13 of Table 1).

### (Example 2)

Preparation of N-{2-chloro-5-[1-(2-iodobenzyloxyimino) ethyl]phenoxy}methyl carbamate (compound 11-9)

### (Process 1)

### Preparation of N-(2-iodobenzyloxy)t-butyl carbamate

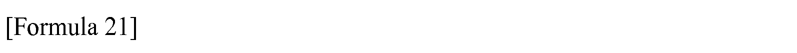

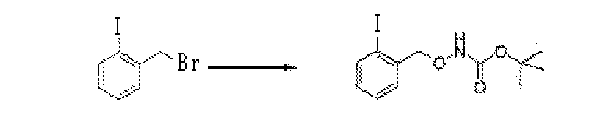

20 ml of acetonitrile, 0.55 g of N-Boc-hydroxylamine and 0.62 g of 1,8-diazabicyclo[5.4.0]-7-undecene (DBU) were added to 1.00 g of 2-iodobenzylbromide, followed by stirring at ambient temperature for one hour. After completion of the reaction, ethyl acetate was added to the reaction mixture, followed by washing with 7% hydrochloric acid and 10% aqueous sodium hydroxide solution in this order. An organic layer was separated, dried over magnesium sulfate, and filtered, and the solvent was then distilled under reduced pressure. 1.08 g of the desired crude N-(2-iodobenzyloxy)t-butyl carbamate (crude yield 92%) was obtained.

### (Process 2)

### Preparation ofN- {2-chloro-5-[1-(2-iodobenzyloxyimino)ethyl]phenoxy}methyl carbamate

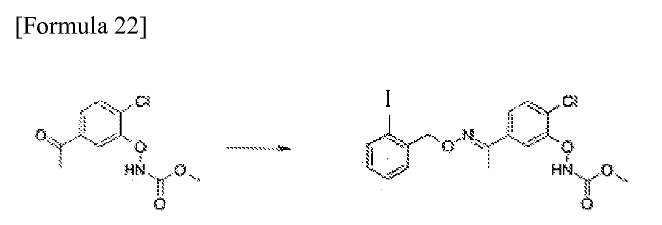

A mixture of 0.30 g ofN-(5-acetyl-2-chlorophenoxy)methyl carbamate) and 0.56 g of crude N-(2-iodobenzyloxy)t-butyl carbamate obtained in (Process 1) was dissolved in 15 ml of dichloroethane, and 1 ml of trifluoroacetic acid was added thereto, followed by stirring at 50°C for one hour. After completion of the reaction, ethyl acetate was added to the reaction mixture, followed by washing with a saturated aqueous sodium bicarbonate solution. An organic layer was separated, dried over magnesium sulfate, and filtered, and the solvent was then distilled under reduced pressure. The resulting residue thus obtained was purified by silica gel column chromatography (eluent; hexane:ethyl acetate=3:1) to obtain 0.34 g of the desired N-{2-chloro-5-[1-(2-iodobenzyloxyimino)ethyl]phenoxy}methyl carbamate (yield 58%, melting point: 121 to 122°C).

### (Example 3)

### Preparation of N-{2-methyl-5-[1-(2-trifluoromethylbenzyloxyimino)ethyl]phenoxy}methyl carbamate (compound 21-24)

### (Process 1)

### Preparation of 4-bromo-2-(4-methoxybenzyloxy)-1-methylbenzene

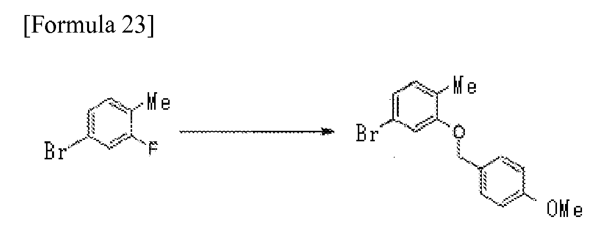

30.0 g of 4-bromo-2-fluorotoluene and 23.1 g of p-methoxybenzylalcohol were dissolved in 240 ml of DMI (dimethyl imidazolidinone), and 6.68 g of sodium hydride was added thereto under ice-cooling, followed by stirring at 110°C for one hour. The reaction solution was cooled to ambient temperature, poured to an aqueous ammonium chloride solution, and extracted with ethyl acetate. The organic layer was dried over magnesium sulfate and filtered, the solvent was distilled under reduced pressure, and the resulting residue thus obtained was purified by silica gel column chromatography (eluent; hexane:ethyl acetate=9:1) to obtain 42.8 g of the desired 4-bromo-2-(4-methoxybenzyloxy)-1-methylbenzene (yield 88%).

### (Process 2) Preparation of 1-[3-(4-methoxybenzyloxy)-4-methylphenyl]ethanone

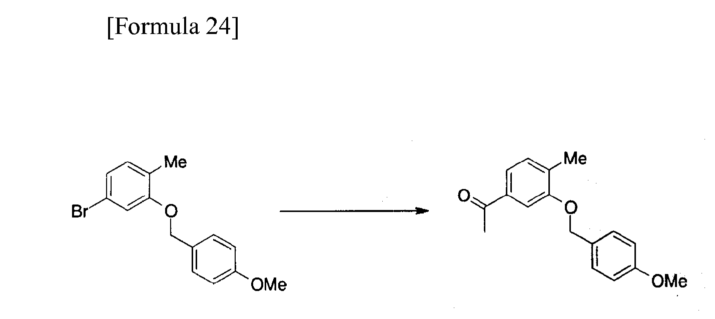

42.8 g of 4-bromo-2-(4-methoxybenzyloxy)-1-methylbenzene was dissolved in 400 ml of THF under a nitrogen atmosphere, 69 ml of n-butyl lithium (2.63 mol/L) was added dropwise thereto at -78°C, the resulting mixture was stirred for 10 minutes, 18.2 g of dimethylacetamide was added dropwise thereto, and the resulting mixture was stirred at -78°C for 30 minutes. After completion of the reaction, an aqueous ammonium chloride solution was added at -78°C and extracted with ethyl acetate. The organic layer was dried over magnesium sulfate and filtered, the solvent was distilled under reduced pressure, and the resulting residue thus obtained was purified by silica gel column chromatography (eluent; hexane:ethyl acetate=9:1 to 5:1) to obtain 24.6 g of the desired 1-[3-(4-methoxybenzyloxy)-4-methylphenyl]ethanone (yield 65%).

### (Process 3) Preparation of 1-(3-hydroxy-4-methylphenyl)ethanone

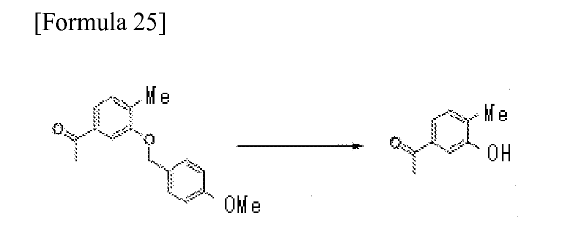

4.74 g of 1-[3-(4-methoxybenzyloxy)-4-methylphenyl]ethanone was dissolved in 50 ml of methylene chloride and 5 ml of trifluoroacetic acid was added, followed by stirring at ambient temperature for one hour. After completion of the reaction, water was added, followed by extracting with methylene chloride. The organic layer was dried over magnesium sulfate and filtered, the solvent was distilled under reduced pressure, the resulting residue thus obtained was purified by silica gel column chromatography (eluent; hexane:ethyl acetate=2:1) to obtain 2.50 g of the desired 1-(3-hydroxy-4-methylphenyl)ethanone (yield 95%). The ¹H-NMR data of the sample thus obtained was as follows.
¹H-NMR (CDCl₃/TMS, δ(ppm)) 7.55(d, H), 7.43(dd, 1H), 7.20(d, 1H), 6.20(s, 1H), 2.58(s, 3H), 2.32(s, 3H)

### (Process 4)

### Preparation of N-(5-acetyl-2-methylphenoxy)methyl carbamate

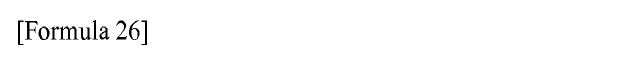

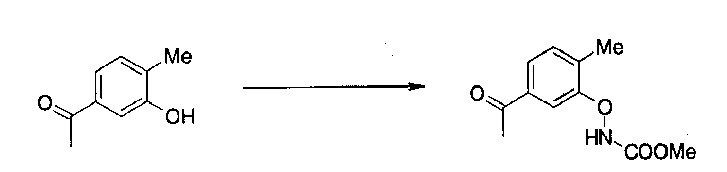

2.61 g of 1-(3-hydroxy-4-methylphenyl)ethanone was dissolved in 30 ml of methanol, 2.35 g of potassium t-butoxide was added thereto, the resulting mixture was stirred at ambient temperature for one hour, and methanol was distilled off under reduced pressure. The resulting residue was dissolved in dimethylformamide, a diethylether solution (ca. 1.5 eq.) of MSH (o-mesythylenesulfonyl hydroxylamine) was added dropwise under ice-cooling and stirred for 10 minutes, and 3.44 g of pyridine and 2.47 g of chloroformic acid methyl ester were added thereto, followed by further stirring for one hour. After completion of the reaction, an aqueous ammonium chloride solution was added thereto, followed by extracting with ethyl acetate. The organic layer was dried over magnesium sulfate and filtered, the solvent was distilled under reduced pressure, and the resulting residue was purified by silica gel column chromatography (eluent; hexane:ethyl acetate=2:1) to obtain 1.16 g of the desired N-(5-acetyl-2-methylphenoxy)methyl carbamate (yield 30%). The phase of sample thus obtained was an oil and ¹H-NMR data thereof was as follows.
¹H-NMR (CDCl₃/TMS, δ(ppm)) 7.79(d, 1H), 7.74(s, 1H), 7.54(dd, 1H), 7.22(d, 1H), 3.82(s, 3H), 2.57(s, 3H), 2.32(s, 3H)

### (Process 5)

### Preparation of N-{2-methyl-5-[1-(2-trifluoromethylbenzyloxyimino) ethyl]phenoxy}methyl carbamate

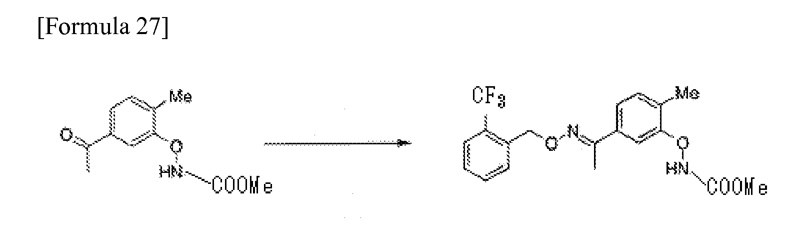

0.15 g ofN-(5-acetyl-2-methylphenoxy)methyl carbamate was dissolved in 15 ml of methanol and 0.30 g of o-(2-trifluoromethylbenzyl)hydroxylamine hydrochloride was added thereto, followed by reflux for 2 hours. After completion of the reaction, ethyl acetate was added thereto, followed by washing with a saturated aqueous ammmonium chloride solution. The organic layer was dried over magnesium sulfate and filtered, and the solvent was distilled under reduced pressure. The resulting residue was purified by silica gel column chromatography(eluent; hexane:ethyl acetate=3:1) to obtain 0.17g of the desired N-{2-methyl-5-[1-(2-trifluoromethylbenzyloxyimino) ethyl]phenoxy}methyl carbamate (yield 63%). The ¹H-NMR data of the sample thus obtained was as follows.
¹H-NMR (CDCl₃/TMS, δ(ppm)) 7.68∼7.62(m, 3H), 7.54(dd, 1H), 7.49(d, 1H), 7.39(dd, 1H), 7.21(dd, 1H), 7.11(d, 1H), 5.44(s, 2H), 3.82(s, 3H), 2.27(s, 3H)

The compound as set forth in Table below was prepared in the same manner as in Examples 1 to 3. The compound No. of Table corresponds to the compound No. of Tables 1 to 28.

**[Table 35]**

| Compound No. | Structural formula | Melting point (°C) |
|---|---|---|
| 1-1 | 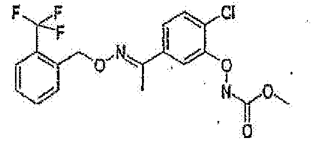 | 85-87 |
| 1-2 | 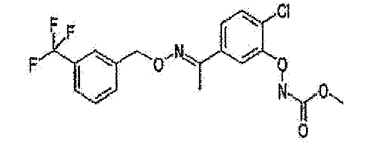 | 75-76 |
| 1-4 | 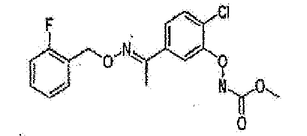 | 115-117 |
| 1-5 | 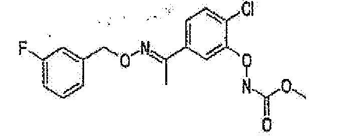 | 90-92 |
| 1-6 | 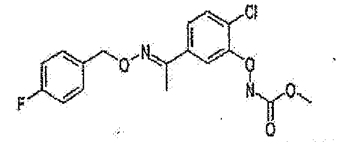 | 110-111 |
| 1-7 | 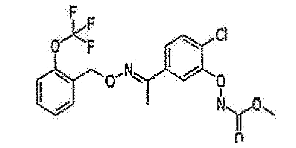 | 83-84 |
| 1-12 | 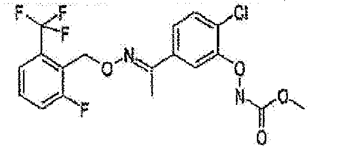 | 100-102 |

**[Table 36]**

| Compound No. | Structural formula | Melting point (°C) |
|---|---|---|
| 1-13 | 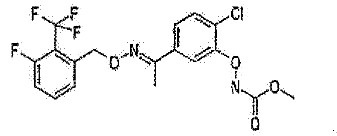 | 104-105 |
| 1-16 | 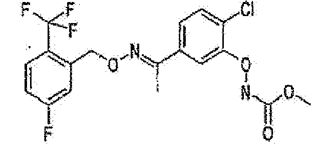 | 106-107 |
| 1-17 | 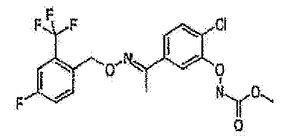 | 103-104 |
| 2-1 | 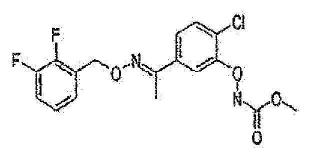 | 129-130 |
| 2-2 | 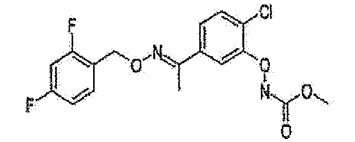 | 139-140 |
| 2-3 | 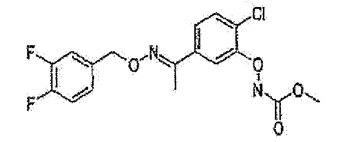 | 130-131 |
| 2-4 | 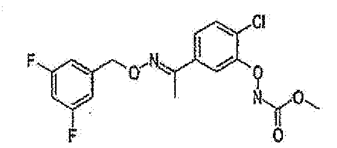 | 140-141 |

**[Table 37]**

| Compound No. | Structural formula | Melting point (°C) |
|---|---|---|
| 2-5 | 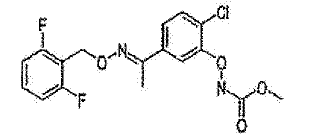 | 138-140 |
| 2-25 | 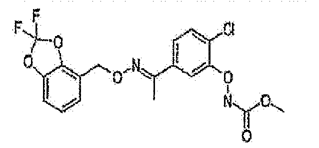 | 115-116 |
| 4-1 | 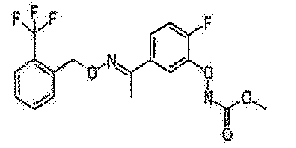 | 79-81 |
| 7-1 | 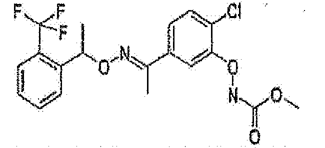 | 146-147 |
| 7-2 | 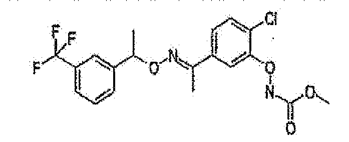 | viscous oil |
| 9-1 | 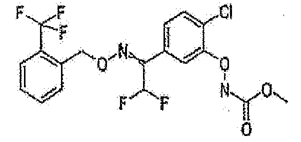 | 97-98 |
| 9-2 | 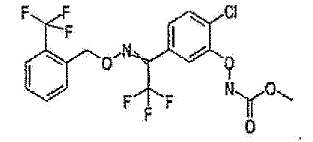 | 111-112 |
| 9-3 | 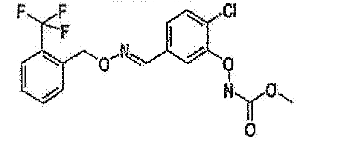 | 99-101 |

**[Table 38]**

| Compound No. | Structural formula | Melting point (°C) |
|---|---|---|
| 9-4 | 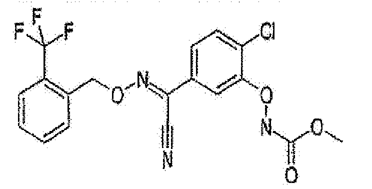 | 132-133 |
| 9-5 | 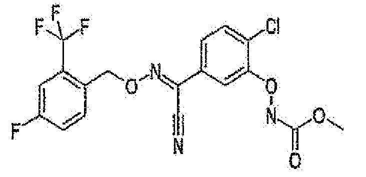 | 149-150 |
| 9-6 | 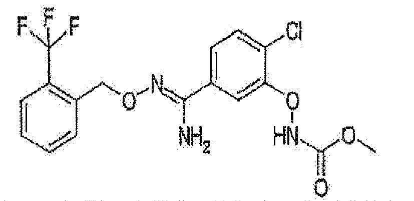 | 165-167 |
| 9-7 | 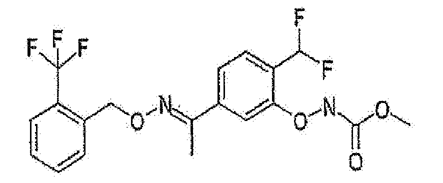 | 101-103 |
| 9-8 | 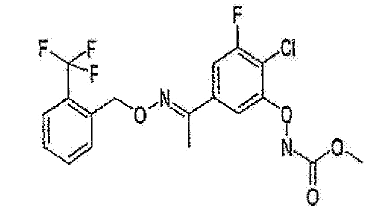 | 114-116 |

**[Table 39]**

| Compound No. | Structural formula | Melting point (°C) |
|---|---|---|
| 9-11 | 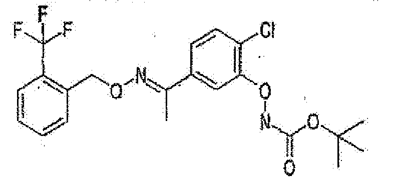 | 109-111 |
| 9-12 | 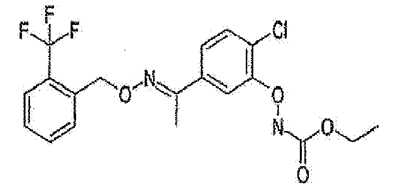 | 104-105 |
| 9-13 | 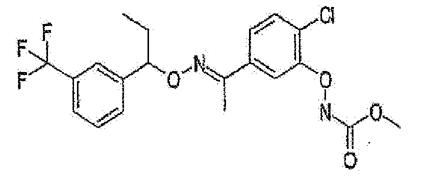 | 24. 2-1. 5221 (Refractive index) |
| 9-14 | 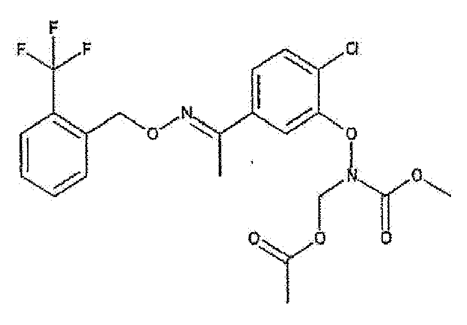 | 84-85 |

**[Table 40]**

| Compound No. | Structural formula | Melting point (°C) |
|---|---|---|
| 9-15 | 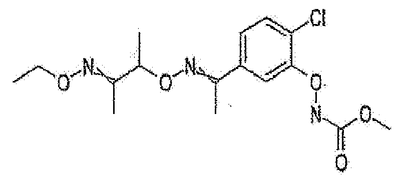 | 23. 4-1. 5190 (Refractive index) |
| 9-16 | 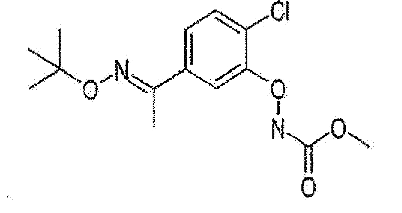 | amorphous |

**[Table 41]**

| Compound | Structural formula No. | Melting point (°C) |
|---|---|---|
| 11-1 | 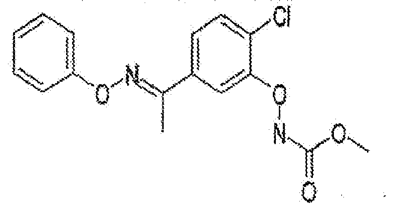 | viscous oil |
| 11-2 | 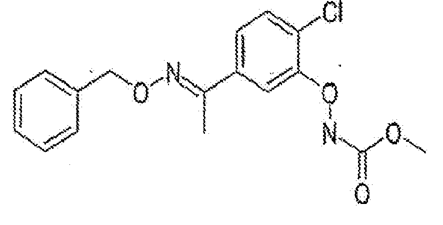 | 119-121 |
| 11-3 | 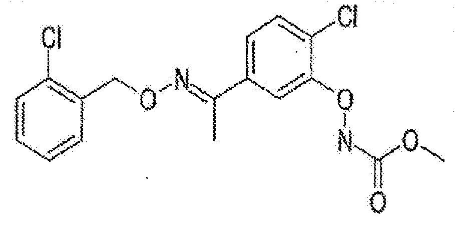 | 138-140 |
| 11-4 | 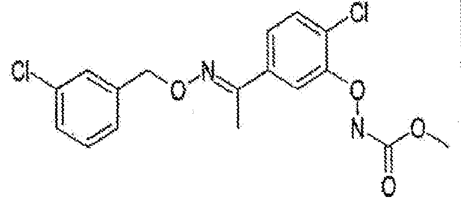 | 125-127 |
| 11-5 | 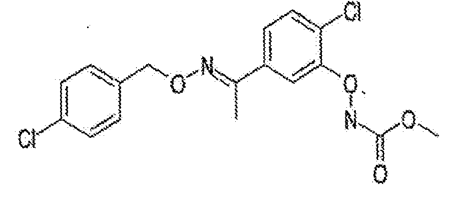 | 111-114 |

**[Table 42]**

| Compound No. | Structural formula | Melting point (°C) |
|---|---|---|
| 11-6 | 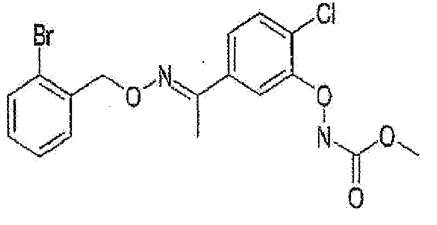 | 143-144 |
| 11-9 | 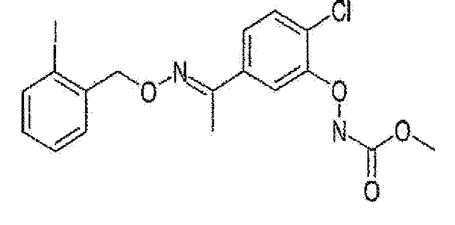 | 121-122 |
| 11-12 | 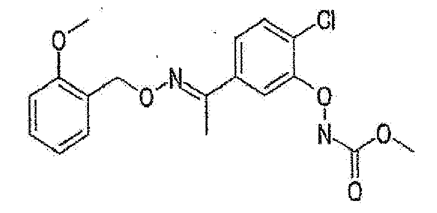 | 118-121 |
| 11-13 | 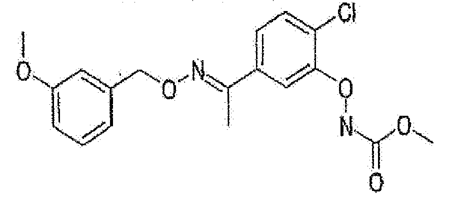 | 93-96 |

**[Table 43]**

| Compound No. | Structural formula | Melting point (°C) |
|---|---|---|
| 11-14 | 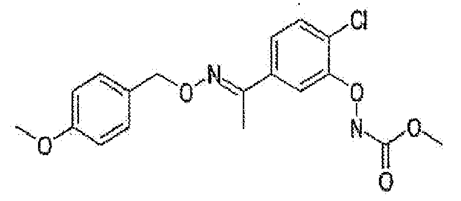 | 98-100 |
| 11-15 | 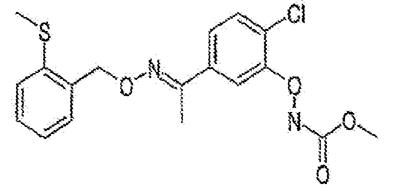 | 140-141 |
| 11-16 | 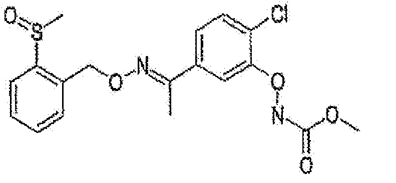 | 135-136 |
| 11-17 | 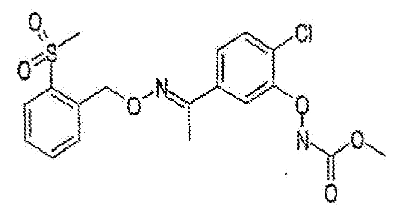 | 125-126 |
| 11-21 | 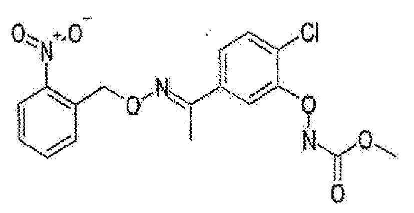 | 163-164 |

**[Table 44]**

| Compound No. | Structural formula | Melting point (°C) |
|---|---|---|
| 11-22 | 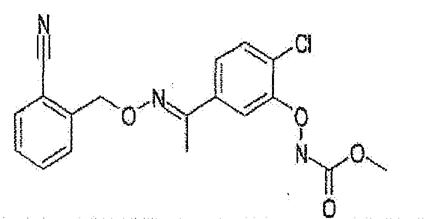 | 147-148 |
| 11-23 | 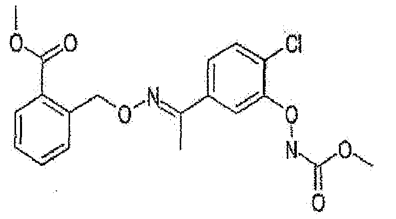 | 147-148 |
| 11-25 | 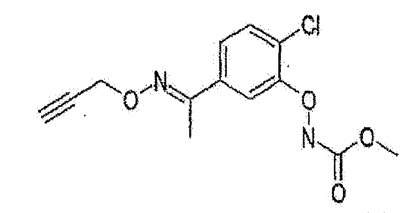 | 98-99 |
| 11-27 | 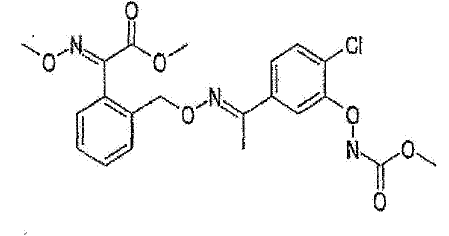 | 119-121 |

**[Table 45]**

| Compound No. | Structural formula | Melting point (°C) |
|---|---|---|
| 21-2 | 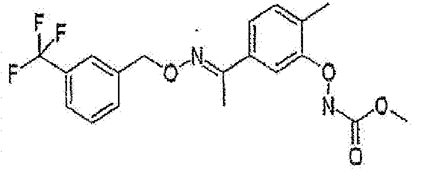 | 64-67 |
| 21-7 | 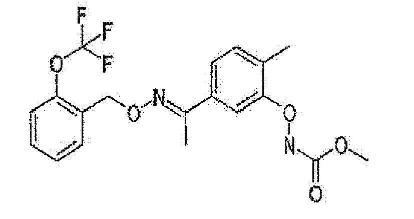 | 59-60 |
| 21-9 | 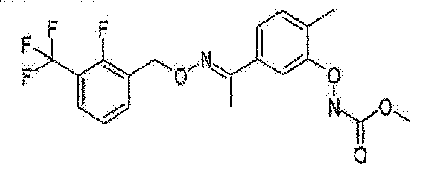 | 94-95 |
| 21-17 | 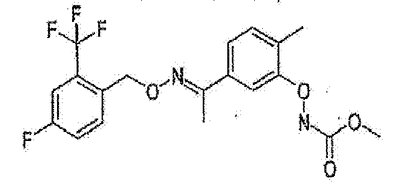 | 80-81 |
| 21-21 | 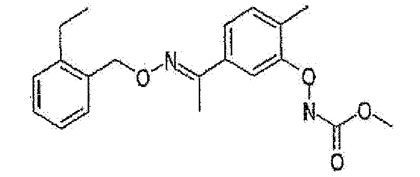 | viscous oil |

**[Table 46]**

| Compound No. | Structural formula | Melting point (°C) |
|---|---|---|
| 21-22 | 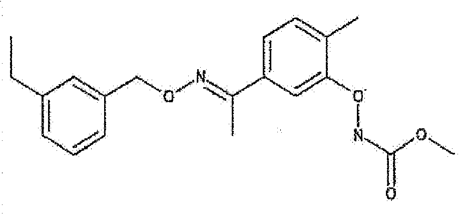 | 80-81 |
| 21-23 | 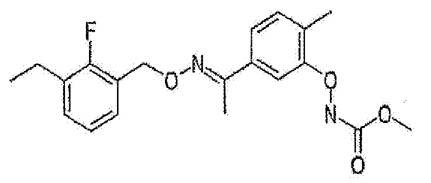 | viscous oil |
| 21-24 | 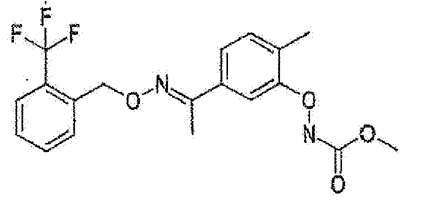 | 82-85 |
| 21-25 | 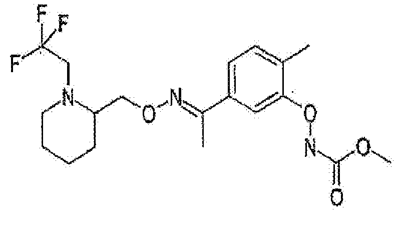 | 22.8-1.5030 (Refractive index) |

**[Table 47]**

| Compound No. | Structural formula | Melting point (°C) |
|---|---|---|
| 21-26 (A) | 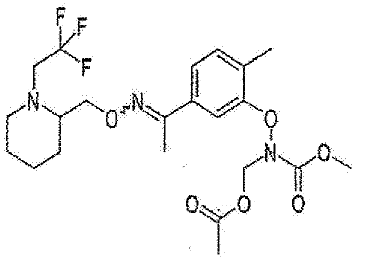 | viscous oil |
| 21-26 (B) | 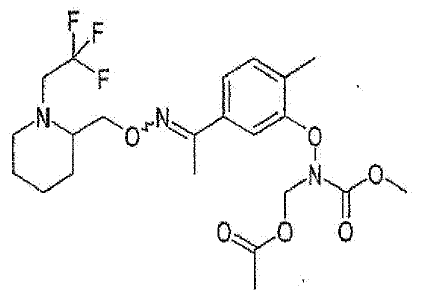 | viscous oil |
| 21-27 | 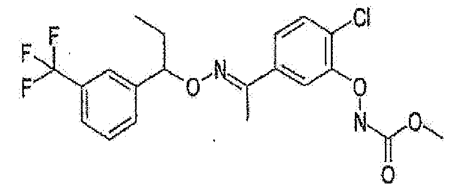 | 24-1.5221 (Refractive index) |
| 21-28 | 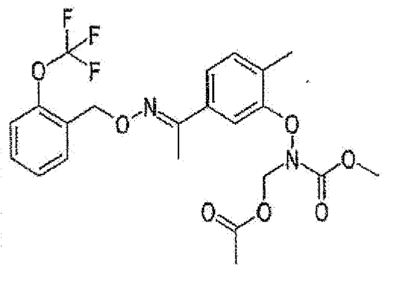 | viscous oil |

**[Table 48]**

| Compound | Structural formula No. | Melting point (°C) |
|---|---|---|
| 27-1 | 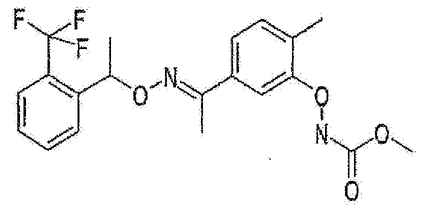 | 95-98 |
| 27-2 | 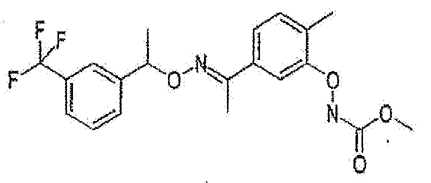 | viscous oil |

**[Table 49]**

| Compound No. | NMR data |
|---|---|
| 7-2, | ¹H-NMR (CDCl₃/TMS, δ(ppm)) 7,66∼7.60(m,3H), 7.53(t,1H) 7.43(s,1H), 7,33(t,1H), 7.14(d,1H), 7,05(d,1H), 5,77(q,1H), 3, 81(s,3H), 2.25(s,3H), 2.23(s,3H), 1.58(d,3H) |
| 9-16 | ¹H-NMR (CDCl₃/TMS, δ (ppm)) 7.78(s,1H), 7,61(dd,1H), 7.3 3∼7,32(m,2H), 3,85(s,3H), 2.17(s,3H), 1,34(s,9H) |
| 11-1 | ¹H-NMR (CDCl₃/IMS,δ(ppm)) 7.79(s,1H), 7.73(s,1H), 7.43∼ 7.24(m,6H), 7.04(t,1H), 3.85(s,3H), 2.42(s,3H) |
| 21-23 | ¹H-NMR (CDCl₃/TMS,δ(ppm)) 7.76(s,1H), -7.48(d,1H), 7.30∼ 7.02(m,5H), 5.29(d,2H), 3.81(s,3H), 2.69(q,2H), 2.25(s,3H), 2(s,3H), 1.24(t,3H) |
| 21-29 | ¹H-NMR (CDCl₃/TMS,δ(ppm)) 7.52(dd,1H), 7.37∼7,11(m,6H ), 5.67(s,2H), 5.31(s,2H), 3,84(s,3H), 2.47(s,3H), 2.23(s,3H), 2, 08(s,3H) |
| 21-26(A) | ¹H-NMR (CDCl₃/TMS,δ(ppm)) 7,32(d,1H), 7.21(dd,1H), 7,13( d,1H), 5.67(s,2H), 4,41∼4.33(m,1H), 3.84(s,3H), 3.27∼3.16(m , 4H), 3.10∼2.81(m,2H), 2.25(s,3H), 2.17(s,3H), 2.10(s,3H), 1. 81∼1.49(m,6H) |
| 21-26(B) | ¹H-NMR (CDCl₃/TMS,δ(ppm)) 7,32(d,1H), 7.21(dd,1H), 7.13( (d,1H), 5,67(s,2H), 4.35(dd,1H), 4,17(dd,1H), 3.85(s,3H), 3,46 21∼26(B) ∼2.92(m,5H), 2.60∼2.51(m,1H), 2.26(s,3H), 2.18(s,3H), 2.11( s,3H), 1.75∼1.38(m,5H) |
| 27-2 | ¹H-NMR(CDCl₃/TMS, (ppm)) 7.65∼7,41 (m,6H), 7.15(dd,1 1H), 7.08(d,1H), 5.41(q,1H), 3.81(s,3H), 2.26(s,3H), 2,25(s,3H), 1,61(d,3H) |
| 27-21 | ¹H-NMR (CDCl₃/TMS, δ (ppm)) 7.93(s,1H), 7.49(d,1H), 7.39( d,1H), 7.30∼7.16(m,4H), 7.09(d,1H), 5.27(s,2H), 3.80(s,3H), 2 76(q,2H), 2.24(s,3H), 2.20(s,3H), 1.25(t,3H) |

Next, several examples of fungicides of the present invention will be described, but additives and addition ratios are not limited thereto and can be widely varied. In addition, the part in Formulation Examples means "part by mass".

| Formulation Example 1 | Wettable powder |
|---|---|
| Compound of the present invention | 40 parts |
| Clay | 48 parts |
| Dioctyl sulfosuccinate sodium | 4 parts |
| Sodium lignosulfonate | 8 parts |

These ingredients were homogeneously mixed and finely ground to obtain a wettable powder containing a 40% active ingredient.

| Formulation Example 2 | Emulsion |
|---|---|
| Compound of the present invention | 10 parts |
| Solvesso 200 | 53parts |
| Cyclohexanone | 26 parts |
| Dodecylbenzene sulfonate calcium | 1 part |
| Polyoxyethylene alkyl aryl ether | 10 parts |

These ingredients were mixed and dissolved to obtain an emulsion concentrate containing a 10% active ingredient.

| Formulation Example 3 | Dusting powder |
|---|---|
| Compound of the present invention | 10 parts |
| Clay | 90 parts |

These ingredients were homogeneously mixed and finely ground to obtain a dusting powder containing a 10% active ingredient.

| Formulation Example 4 | Granules |
|---|---|
| The compound of the present invention | 5 parts |
| Clay | 73 parts |
| Bentonite | 20 parts |
| Dioctyl sulfosuccinate sodium | 1 part |
| Potassium phosphate | 1 part |

These ingredients were ground and mixed well, then mixed well with water, and dry-granulated to obtain a granules containing a 5% active ingredient.

| Formulation Example 5 | Suspending agent |
|---|---|
| The compound of the present invention | 10 parts |
| Polyoxyethylene alkyl aryl ether | 4parts |
| Sodium polycarbonate | 2 parts |
| Glycerine | 10 parts |
| Xanthan gum | 0.2 parts |
| Water | 73.8 parts |

These ingredients were mixed and wet-ground to a particle diameter of 3 microns or less to obtain a suspending agent containing a 10% active ingredient.

| Formulation Example 6 | Water dispersible granule |
|---|---|
| The compound of the present invention | 40 parts |
| Clay | 36 parts |
| Potassium chloride | 10 parts |
| Alkylbenzene sulfonate sodium | 1 part |
| Sodium lignosulfonate | 8 parts |
| Formaldehyde condensate of alkylbenzene sulfonate sodium | 5 parts |

These ingredients were homogeneously mixed, finely ground and, homogeneously mixed with a suitable amount of water to mold a clay. The clay molding was granulated and dried to obtain a wettable powder containing a 40% active ingredient.

The fungicide of the present invention thus obtained will be illustrated in Experimental Examples.

### (Experimental Example 1) Venturia inaequalis control test for apples

An emulsifiable concentrate according to the compound of the present invention as an active ingredient (concentration: 100 ppm) was sprayed to an apple seedling (cv. "Ralls Janet", 3 to 4 leaf stage) grown in a bisque-fired pot. The seedling was naturally dried at ambient temperature, and *Venturia inaequalis* conidium was inoculated thereon and maintained at a light-dark (12 hr/12 hr) cycle indoor at 20°C for 2 weeks at a high humidity. The leaf lesion appearance of the seedling was compared with a non-treated seedling to evaluate control effects.

As a result, the following compounds exhibited a high preventive value of 75% or higher.
The compound numbers (the compound numbers correspond to the compound number of Tables; the same will be applied below): 1-1, 1-2, 1-4, 1-5, 1-6, 1-7, 1-12, 1-13, 1-16, 1-17, 2-1, 2-2, 2-3, 2-4, 2-5, 2-25, 4-1, 7-1, 7-2, 9-1, 9-2, 9-3, 9-4, 9-6, 9-7, 9-8, 9-12, 9-14, 9-15, 9-16, 11-1, 11-2, 11-3, 11-4, 11-5, 11-6, 11-9, 11-12, 11-13, 11-14, 11-15, 11-17, 11-22, 11-23, 11-25, 11-27, 21-2, 21-7, 21-9, 21-17, 21-21, 21-22, 21-23, 21-24, 21-25, 21-26(A), 21-26(B), 21-27, 21-28, 27-1, 27-2.

### (Experimental Example 2) Erysiphe graminis f. sp. tritici control test for wheats

A wettable powder according to the compound of the present invention as an active ingredient (concentration: 100 ppm) was sprayed to a wheat seedling (cv. "Chihoku" 1.0-1.2 leaf stage) grown in a biscuit-fired pot. The leaf was dried in air, and *Erysiphe graminis f. sp. tritici* conidium was inoculated thereon by conidial-detachment and maintained in hot house at 22 to 25°C for 7 days. The leaf lesion appearance of the seedling was compared with a non-treated seedling to evaluate control effects.

As a result, the following compounds exhibited a high preventive value of 75% or higher.
Compound number: 1-1, 1-2, 1-4, 1-5, 1-6, 1-7, 1-12, 1-13, 1-16, 1-17,2-1,2-25, 4-1, 7-1, 7-2, 9-1, 9-2, 9-3, 9-7, 9-8, 9-14, 9-15, 9-16, 11-2, 11-3, 11-4, 11-5, 11-9, 11-13, 11-14, 11-15, 11-16, 11-17, 11-27, 21-2, 21-7, 21-9, 21-17, 21-21, 21-22, 21-23, 21-24, 21-27, 21-28, 27-1, 27-2.

### (Experimental Example 3) Puccinia recondita control test for wheats

A wettable powder according to the compound of the present invention as an active ingredient (concentration: 100 ppm) was sprayed to a wheat seedling (cv. "Horin 6 1 ", 1.0 to 1.2 leaf stage) grown in a bisque-fired pot. The leaf was dried in air, and *Puccinia recondita* uredospore was inoculated thereon by conidial-detachment and maintained in hot house at 22 to 25°C for 10 days. The leaf lesion appearance of the seedling was compared with a non-treated seedling to evaluate control effects.

As a result, the following compounds exhibited a high preventive value of 75% or higher.
Compound number: 1-1, 1-2, 1-4, 1-5, 1-6, 1-7, 1-12, 1-13, 1-16, 1-17,2-1,2-2, 2-5, 2-25, 4-1, 7-1, 7-2, 9-1, 9-2, 9-3, 9-7, 9-8, 9-14, 9-15, 9-16, 11-1, 11-2, 11-3, 11-4, 11-5, 11-9, 11-12, 11-13, 11-15, 11-17, 11-23, 11-25, 11-27, 21-2, 21-7, 21-9, 21-17, 21-21, 21-22, 21-23, 21-24, 21-25, 21-26(A), 21-26(B), 21-27, 21-28, 27-1, 27-2.

### Industrial Applicability

The oxime ether derivative and a salt thereof of the present invention are novel compounds, can be industrially advantageously prepared, and are useful as an active ingredient of fungicide for agriculture and horticulture which exhibits potent effects and can be safely thus used.
The fungicide for agriculture and horticulture of the present invention exhibits superior control effects, is free of phytotoxicity or causing contamination to plants, and has low toxicity or environmental effects in regard to humans, livestock or fish.

## Claims

1. An oxime ether derivative represented by Formula (I) below or a salt thereof: 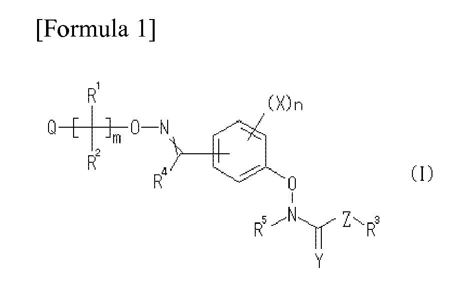 (wherein
X represents a halogen atom, a C1-C20 alkyl group or a C1-C20 alkoxy group, and in the case where n is 2 or more, adjacent X may be bonded together to form a ring;
R¹ and R² each independently represent a hydrogen atom, a halogen atom, a C1-C20 alkyl group or a C3-C10 cycloalkyl group. R¹ and R² may be bonded together to form a ring;
R³ represents a hydrogen atom, a C1-C20 alkyl group, a C2-C20 alkenyl group, a C2-C20 alkynyl group or a C3-C10 cycloalkyl group;
R⁴ represents a hydrogen atom, a C1-C20 alkyl group, a C2-C20 alkenyl group, a C2-C20 alkynyl group, a C3-C10 cycloalkyl group, a cyano group or an amino group;
R⁵ represents a hydrogen atom, a C1-C20 alkyl group, a C2-C20 alkenyl group, a C2-C20 alkynyl group, an acyl group, a C1-C20 alkylsulfonyl group, or a C1-C20 arylsulfonyl group;
Y represents an oxygen atom or a sulfur atom;
Z represents a single bond, an oxygen atom, a sulfur atom, or a group represented by NR⁶ (in which R⁶ represents a hydrogen atom or a C1-C30 alkyl group; and
Q represents a C1-C20 alkyl group, an aryl group, a saturated heterocyclic ring, or a group represented by Formula (III) or Formula (IV).) 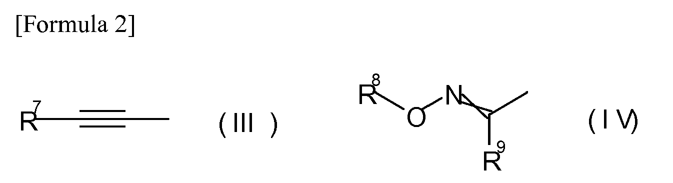 (wherein
R⁷ represents a hydrogen atom, a halogen atom, or a C1-C20 alkyl group; R⁸ represents a hydrogen atom or a C1-C20 alkyl group;
R⁹ represents a hydrogen atom or a C1-C20 alkyl group;
m represents an integer of 0 to 8, and in the case where m is 2 or more, each of R¹ and R² may independently be identical or different; and
n represents an integer of 0 to 4, and in the case where n is 2 or more, X may be identical or different.)

2. The oxime ether derivative or a salt thereof according to claim 1, represented by the following Formula (V): 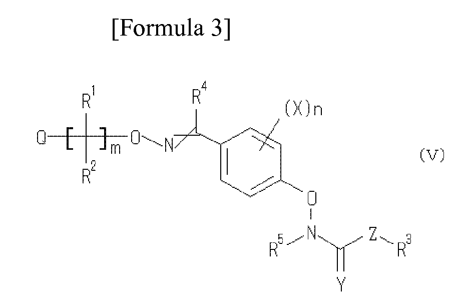 (wherein X, R¹ to R⁵, Y, Z, Q, m and n are defined as above.)

3. The oxime ether derivative or a salt thereof according to claim 1, represented by the following Formula (VI): 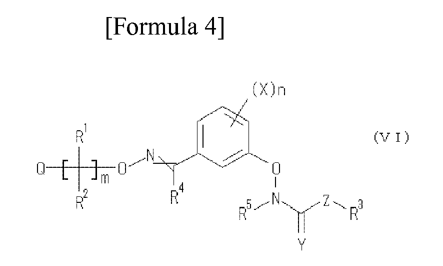 (wherein X, R¹ to R⁵, Y, Z, Q, m and n are defined as above)

4. A fungicide for agriculture and horticulture containing at least one of the oxime ether derivative or a salt thereof according to any one of claims 1 to 3, as an active ingredient.
